# FASCICULE DE BREVET EUROPEEN

(11) **EP 0 905 118 B1**
(45) Date de publication et mention de la délivrance du brevet: **16.08.2001**
(21) Numéro de dépôt: 98402373.9
(22) Date de dépôt: 25.09.1998
(51) Int. Cl.: C07C 57/50, C07C 69/618, C07D 333/38, A61K 31/19, A61K 31/22, A61K 31/38, A61K 7/06, A61K 7/48

(54) **Dérivés insaturés en position 4 du 6-tert-butyl-1,1-diméthylindane et leur utilisation en médicine humaine ou vétérinaire ainsi qu'en cosmétique**
Ungesättigte in 4-Stellung substituierte 6-tert-Butyl-1,-Dimethylindan-Derivate und deren Verwendung in der Human- und Veterinärmedizin
Unsaturated 6-tert-butyl-1,1-dimethylindane derivates substituted in the 4-position and their use in human and veterinary medicine as well as in cosmetics

(30) Priorité: 25.09.1997 FR 9711946
(43) Date de publication de la demande: 31.03.1999
(73) Titulaire: Galderma Research & Development, S.N.C., 06560 Valbonne (FR)
(72) Inventeur: de Lera, Angel, 36211 Vigo (ES); Dominguez, Beatriz, 36205 Vigo, Pontevedra (ES)
(74) Mandataire: Stalla-Bourdillon, Bernard

(56) Documents cités:
- EP-A- 0 595 197
- FR-A- 2 390 428
- FR-A- 2 601 670

## Description

La présente invention a pour objet, de nouveaux dérivés insaturés en position -4 du 6-tert-butyl-1,1-diméthylindane, leur procédé de préparation et leur utilisation en médecine humaine ou vétérinaire ainsi qu'en cosmétique.

Il est bien connu que l'acide all-trans rétinoïque (AtRA) et certains analogues synthétiques (les rétinoïdes) jouent un rôle fondamental dans la prolifération et la différenciation cellulaire. Ces propriétés pharmacologiques confèrent aux rétinoïdes un intérêt dans le traitement d'affections dermatologiques, telles que l'acné ou le psoriasis. En outre, ces composés ont des applications en oncologie dans le traitement et la prévention de certains cancers.

Les composés selon l'invention qui sont des composés synthétiques également du type rétinoïde présentent une activité dans les domaines de la différenciation et de la prolifération cellulaire. En conséquence, ces composés peuvent être utilisés dans le traitement topique et systémique des affections dermatologiques liées à un désordre de la kératinisation, des affections dermatologiques (ou autres) à composante inflammatoire, virale et/ou immunoallergique et des proliférations dermiques ou épidermiques, qu'elles soient bénignes ou malignes. Ces composés peuvent être utilisés en outre, dans le traitement des maladies de dégénérescence du tissu conjonctif, pour lutter contre le vieillissement de la peau, qu'il soit photoinduit ou chronologique et traiter les troubles de la cicatrisation. Ils trouvent enfin une application dans le domaine ophtalmologique, notamment dans le traitement des cornéopathies.

On peut également les utiliser dans les compositions cosmétiques pour l'hygiène corporelle ou capillaire.

Les composés selon l'invention peuvent être représentés

Parmi les analogues synthétiques appartenant à la classe des rétinoïdes, on peut notamment citer ceux décrits dans la demande FR-A-2 601 670 qui sont des dérivés bicycliques, en particulier des dérivés aromatiques du tétrahydronaphyle ainsi que ceux décrits dans la demande FR-A-2 390 428 qui sont des composés aromatiques polyéniques, en particulier des dérivés polyéniques de l'indanyle. par la formule générale (I) suivante : 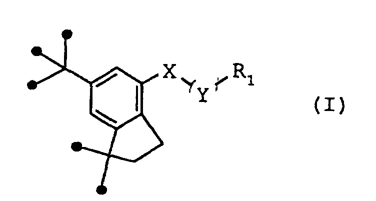 dans laquelle :
X représente :
   (i) soit un radical divalent de formule suivante : 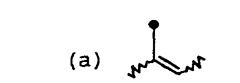 et Y représente alors un radical divalent de formule suivante : 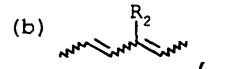
   (ii) soit un radical divalent de formule : 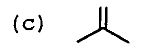 et Y représente alors soit un radical divalent correspondant au radical divalent de formule (b) ci-dessus soit l'un des radicaux divalents de formule suivante : 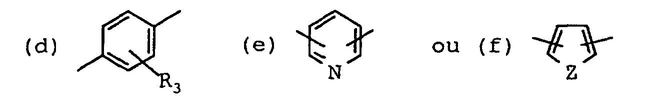
Z étant ―O―, -S- ou 〉N-R₃
R₁ représente ―CH₃, - (CH₂)ₚ-OR₄ , - (CH₂)ₚ-COR₅, ou -S(O)ₜ-R₆,
p étant 0, 1, 2 ou 3,
t étant 0, 1 ou 2,
R₂ représente H ou alkyle linéaire ou ramifié en C₁-C₆,
R₃ représente H, alkoxy en C₁-C₆ ou -OCOR₇,
R₄ représente H, alkyle linéaire ou ramifié en C₁-C₆, ―COR₇, aryle, aralkyle, mono ou polyhydroxyalkyle, ou un radical polyéther,
R₅ représente H, alkyle linéaire ou ramifié en C₁-C₆, ―OR₈ ou 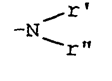
R₆ représente H ou alkyle linéaire ou ramifié en C₁-C₆,
R₇ représente alkyle linéaire ou ramifié en C₁-C₆,
R₈ représente H, alkyle, alkényle, alkynyle, aryle, aralkyle, mono- ou polyhydroxyalkyle, un reste de sucre ou d'acide aminé,
r' et r", identiques ou différents, représentent H, alkyle linéaire ou ramifié en C₁-C₆, ―COR₇, aryle, un reste de sucre, un reste d'acide aminé ou r' et r", pris ensemble, forment un hétérocycle et les sels des composés de formule (I) lorsque R₁ représente une fonction acide carboxylique et les isomères géométriques et optiques des composés de formule (I).

Lorsque les composés selon l'invention se présentent sous forme d'un sel, il s'agit de préférence d'un sel d'un métal alcalin ou alcalino-terreux ou encore de zinc ou d'une amine organique.

Selon l'invention, le radical alkyle inférieur, linéaire ou ramifié, en C₁-C₆, est choisi de préférence, parmi les radicaux méthyle, éthyle, propyle, isopropyle, tertio butyle et n-hexyle.

Par radical alkyle, on entend un radical, linéaire ou ramifié, en C₁-C₂₀, de préférence les radicaux méthyle, éthyle, isopropyle, butyle, tertiobutyle, 2-éthyl-hexyle, octyle, dodécyle, hexadécyle et octadécyle.

Le radical alkoxy inférieur est un radical ayant de 1 à 6 atomes de carbone de préférence les radicaux méthoxy, éthoxy, isopropoxy et butoxy.

Par radical alkényle on entend un radical ayant de préférence de 2 à 5 atomes de carbone, linéaire ou ramifié, et présentant une ou deux insaturation(s) éthylénique(s) tel que le radical allyle.

Par radical alkynyle on entend un radical ayant de 3 à 6 atomes de carbone, comportant 1 ou 2 triple(s) liaison(s) tel que les radicaux 2-propynyle, 2-butynyle et 2,4-hexadiynyle.

Le radical de formule ―COR₇ (R₇ étant alkyle linéaire ou ramifié, en C₁-C₆ ou acyle est de préférence choisi parmi les radicaux acétyle, propionyle ou pivaloyle.

Par radical monohydroxyalkyle, on entend un radical ayant de préférence de 2 à 4 atomes de carbone, de préférence les radicaux 1-hydroxyéthyle, 2-hydroxypropyle, 3-hydroxypro-pyle ou 4-hydroxybutyle.

Par radical polyhydroxyalkyle, on entend un radical ayant de préférence de 3 à 6 atomes de carbone et de 2 à 5 groupes hydroxyles tel que les radicaux 2,3-dihydroxypropyle, 2,3,4-trihydroxybutyle, 2,3,4,5-tétrahydroxypentyle ou le reste du pentaérythritol.

Par radical polyéther, on entend un radical ayant de 2 à 6 atomes de carbone et de 1 à 3 atomes d'oxygène ou de soufre tel que Les radicaux méthoxyméthyléther, méthoxyéthoxyméthyléther ou méthylthiométhyléther.

Par radical aryle, on entend de préférence le radical phényle, éventuellement substitué par au moins un atome d'halogène tel que Br, Cl ou I, une fonction hydroxyle, une fonction nitro, un groupe méthoxy ou une fonction amine éventuellement substitué.

Par radical aralkyle, on entend de préférence le radical benzyle ou phénéthyle éventuellement substitué par au moins un atome d'halogène tel que défini ci-dessus, une fonction hydroxyle, une fonction nitro, ou un groupe méthoxy.

Par reste d'acide aminé, on entend notamment un reste dérivant de la lysine, de la glycine ou de l'acide aspartique.

Par reste de sucre, on entend un reste dérivant du glucose, du galactose ou du mannose ou bien encore de l'acide glucuronique.

Par hétérocycle, on entend de préférence un radical pipéridino, morpholino, pyrrolidino ou pipérazino, ce dernier étant éventuellement substitué en position 4 par un alkyle inférieur en C₁-C₆ ou un mono- ou polyhydroxyalkyle tels que définis ci-dessus.

Selon une première forme de réalisation préférée, les composés selon l'invention répondent à la formule générale suivante : 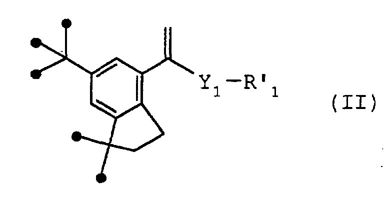 dans laquelle :
Y₁ représente l'un des radicaux divalents de formule : 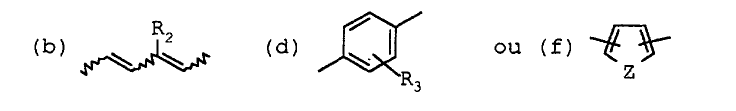
R₂, R₃ et Z ayant les mêmes significations que celles données pour la formule (I) ci-dessus,
R'₁ représente, -(CH₂)ₚ,―COOR'₈ ,p' étant 0 et R'₈ représentant H ou alkyle linéaire ou ramifié, en C₁-C₆.

Selon une deuxième forme de réalisation préférée, les composés selon l'invention répondent à la formule générale suivante : 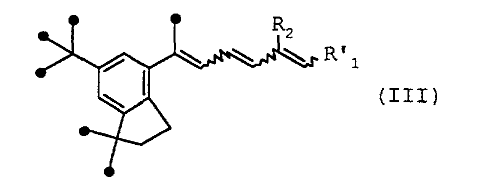 dans laquelle :
R₂ représente H ou alkyle linéaire ou ramifié en C₁-C₆, et
R'₁ représente, -(CH₂)ₚ,―COOR'₈, p' étant 0 et R'₈ étant H ou alkyle linéaire ou ramifié en C₁-C₆.

Parmi les composés répondant aux formules (I), (II) et (III) ci-dessus, on peut notamment citer les exemples suivants :
- (2E,4E)-6-[4-(6*-tert*-butyl-1,1-diméthylindanyl)]-3-méthylhepta-2,4,6-triènoate de méthyle,
- Acide (2E,4E)-6-[4-(6-*tert*-butyl-1,1-diméthylindanyl)]-3-méthylhepta-2,4,6-triènoïque,
- (2E,4E)-6-[4-(6*-tert*-butyl-1,1-diméthylindanyl)]-hepta-2,4,6-triènoate de méthyle,
- Acide (2E,4E)-6-[4-(6*-tert*-butyl-1,1-diméthylindanyl)]-hepta-2,4,6-triènoïque,
- (2E,4E,6E)-7-[4-(6*-tert*-butyl-1,1-diméthylindanyl)]-3,7-diméthylhepta-2,4,6-triènoate de méthyle,
- Acide (2E,4E,6E)-7-[4-(6*-tert*-butyl-1,1-diméthylindanyl)] -3,7-diméthylhepta-2,4,6-triènoïque,
- (2E,4E,6E)-7-[4-(6*-tert*-butyl-1,1-diméthylindanyl)]-7-méthylhepta-2,4,6-triènoate de méthyle,
- Acide (2E,4E,6E)-7-[4-(6*-tert*-butyl-1,1-diméthylindanyl)]-7-méthylhepta-2,4,6-triènoïque,
- 4-[1-[4-(6*-tert*-butyl-1,1-diméthylindanyl)]éthènyl]benzoate d'éthyle,
- Acide 4-[1-[4-(6*-tert*-butyl-1,1-diméthylindanyl)]éthènyl]benzoïque,
- Acide 5-[1-[4-(6*-tert*-butyl-1,1-diméthylindanyl)]éthènyl]-2-thiophènecarboxylique.

La présente invention a également pour objet le procédé de préparation des composés de formule (I) selon les schémas réactionnels des Tableaux A et B.

En se référant au Tableau A, le 4-acétyl-6*-tert*-butyl-1,1-diméthylindane (1) est transformé intermédiairement en 6-*tert*-butyl-4-éthènyl-1,1-diméthylindane par traitement avec du lithium diisopropylamide (LDA) puis est traité par un chlorophosphate d'alkyle tel que le chlorophosphate de diéthyle. Le phosphate mixte résultant intermédiaire est ensuite à nouveau traité par du LDA et conduit ainsi au 6*-tert*-butyl-4-éthynyl-1,1-diméthylindane (2).

Ce dernier peut être alors transformé en iodure vinylique (3) par action de l'iodure ou du chlorure de triméthylsilyle ou être transformé en iodure propylénique (4) par une méthylalumination médiée par un complexe du zirconium (complexe bis(cyclopentadiènyl)zirconium dichlorure) suivie d'un échange entre l'aluminium et l'iode selon les conditions décrites par A. Tollado et al. Tetrahedron, **1995,** vol. 5 pages 2435-2454.

Les dérivés iodés (3) et (4) sont ensuite utilisés dans une réaction de cross-couplage catalysée par du palladium (complexe tris(dibenzylidèneacétone) palladium) avec un composé organostannique polyénique de formule (5) pour conduire aux composés des formules (Ia) et (Ib). Le composé organostannique (5) peut être remplacé par un acide boronique polyénique et dans ce cas, la réaction est réalisée dans les conditions de cross-couplage de type Suzuki, modifiée par Kishi. Selon ces conditions, le milieu contient de l'hydroxyde de thallium et le catalyseur est le Pd(PPh₃)₄, celles-ci étant décrites de façon détaillée dans Synthesis **1995**, No. 3, p. 285.

En se référant maintenant au Tableau B, les composés de formule (Ic) peuvent être obtenus à partir de l'iodure vinylique (3) du Tableau A par condensation avec un dérivé aryle boronique (6) en présence de palladium tétrakistriphénylphosphine selon les conditions décrites ci-dessus dans Synthesis.

Lorsque R₁, dans les composés selon l'invention représente -COOH, ceux-ci sont préparés en protégeant la fonction acide carboxylique par un groupe protecteur de type alkyle, allylique ou *tert*-butylique.

Le passage à la forme libre peut être effectué :
- dans le cas d'un groupe protecteur alkyle au moyen de soude ou d'hydroxyde de lithium dans un solvant alcoolique tel le méthanol ou dans le THF ;
- dans le cas d'un groupe protecteur allylique au moyen d'un catalyseur tel certains complexes de métaux de transition en présence d'une amine secondaire telle la morpholine ;
- dans le cas d'un groupe protecteur de type tert-butylique au moyen d'iodure de triméthylsilane.

Lorsque R₁ dans les composés selon l'invention représente -OH, ceux-ci peuvent être obtenus à partir de l'acide correspondant par réduction en présence d'hydrure double de lithium et d'aluminium.

Lorsque R₁ représente 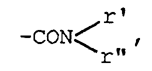 les composés peuvent être obtenus par transformation de l'acide correspondant en chlorure d'acide par exemple avec du chlorure de thionyle puis réaction avec l'ammoniaque ou une amine appropriée.

Les composés selon l'invention se lient aux récepteurs RXRs et possèdent une activité agoniste sur ces récepteurs.

Les propriétés de binding et de transactivation comme agonistes RXR sont déterminées par des méthodes connues telles que par exemple, selon Levin et al. Nature, **1992,** 355, 359-361 ; Allenby et al. Proc. Natl. Acad. Sci. **1993,** 90, 30-4 ; Allenby et al. *J*. Biol. Chem., **1994,** 269, 16689-16695.

L'activité agoniste RXR est aussi déterminée par le test tel que décrit dans la demande de brevet EP 749 752. Ce test comprend les étapes suivantes :
(i) on applique topiquement sur une partie de la peau d'un mammifère une quantité suffisante d'un composé qui est un ligand actif d'au moins un récepteur de la superfamille des récepteurs nucléaires stéroïdiens/thyroïdiens autre qu'un ligand spécifique des récepteurs RXRs et pouvant s'hétérodimériser avec les RXRs, comme par exemple une molécule agoniste RAR,
(ii) on administre par voie systémique ou topique sur cette même partie de la peau du mammifère, avant, pendant ou après l'étape (i) une molécule susceptible de présenter une activité agoniste des RXRs,
(iii)on évalue alors la réponse pharmacologique sur la partie de la peau ainsi traitée du mammifère. Cette réponse consiste en un épaississement de l'oreille. Ainsi, la réponse d'une application topique sur l'oreille d'un mammifère d'un agoniste PAR sera-t-elle potentialisée par d'administration topique ou systémique d'un agoniste des récepteurs RXRs.

La présente invention a également pour objet à titre de médicament, les composés de formule (I) tels que définis ci-dessus.

Les composés selon l'invention conviennent particulièrement bien dans le domaines des traitements suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de troubles de la kératinisation, notamment les ichthyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de troubles de la kératinisation.
4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou toute autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux.
12) Dans le traitement d'affections inflammatoires telles que l'arthrite.
13) Dans le traitement de toute affection d'origine virale au niveau cutané ou général.
14) Dans la prévention ou le traitement de l'alopécie.
15) Dans le traitement d'affections dermatologiques ou générales à composante immunologique.
16) Dans le traitement d'affections du système cardiovasculaire telles que l'artériosclérose, l'hypertension, le diabète non-insulino dépendant ainsi que l'obésité.

Dans les domaines thérapeutiques mentionnés ci-dessus, les composés selon l'invention peuvent être avantageusement employés en combinaison avec d'autres rétinoïdes connus, avec les vitamines D ou leurs dérivés, avec des corticostéroïdes ou oestrogènes, ou employés en association avec des anti-radicaux libres, avec des α-hydroxy ou α-céto acides ou leurs dérivés, ou encore avec des bloqueurs de canaux potassiques.

Par vitamines D ou leurs dérivés on entend par exemple les dérivés de la vitamine D₂ ou D₃ et en particulier la 1,25-dihydroxyvitamine D₃.

Par anti-radicaux libres on entend par exemple l'α-tocophérol, la Super Oxyde Dismutase (SOD), l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique, ascorbique et les dérivés d'acide salicylique ou leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

Les compositions médicamenteuses sont plus particulièrement destinées notamment au traitement des affections mentionnées ci-dessus et sont caractérisées par le fait qu'elles contiennent, dans un support pharmaceutiquement acceptable, au moins un composé de formule (I) l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Les composés selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indic:ation clinique.

Par voie oculaire, ce sont principalement des collyres.

Les compositions pour la voie topique ou oculaire contiennent au moins un composé de formule (I) telle que définie ci-dessus, l'un de ses isomères optiques ou géométriques ou un de ses sels, à une concentration de préférence comprise entre 0,001 et 5 % par rapport au poids total de la composition.

Les composés de formule (I) selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, tout comme dans le domaine thérapeutique, les composés selon l'invention peuvent être avantageusement employés en combinaison avec d'autres rétinoïdes connus, avec les vitamines D ou leurs dérivés, ou avec des corticostéroïdes, ou encore en association avec des anti-radicaux libres, avec des α-hydroxy ou α-céto acides ou leurs dérivés, ou encore avec des bloqueurs de canaux potassiques.

Les compositions cosmétiques selon la présente invention contiennent, dans un véhicule cosmétiquement acceptable, au moins un composé de formule (I) l'un de ses isomères optiques ou géométriques ou l'un de ses sels, celles-ci se présentant notamment sous forme d'une crème, d'un lait, d'une lotion, d'un gel, de microsphères ou nanosphères ou de vésicules lipidiques ou polymériques, d'un savon ou d'un shampooing.

La concentration en composé de formule (I) dans les compositions cosmétiques est de préférence comprise entre 0,001 et 3 % en poids.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants ; des agents dépigmentants tels que l'hydroquinone, l'acide azélaïque, l'acide caféique ou l'acide kojique ; des émollients ; des agents hydratants comme le glycérol, du polyéthylèneglycol 400, de la thiamorpholinone et ses dérivés ou de l'urée ; des agents antiséborrhéiques ou antiacnéiques, tels que la S-carboxyméthylcystéine, la S-benzyl-cystéamine, leurs sels et leurs dérivés, ou du peroxyde de benzoyle ; des antibiotiques comme l'érythromycine et ses esters, la néomycine, la clindamycine et ses esters, les tétracyclines ; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione) ; des agents anti-inflammatoires non stéroïdiens ; des caroténoïdes et, notamment le β-carotène ; des agents anti-psoriatiques tels que l'anthraline et ses dérivés et enfin, les acides eicosa-5,8,11,14-tétraynoïque et eicosa-5,8,11-triynoïque, leurs esters et amides.

Les compositions selon l'invention peuvent également contenir des agents d'amélioration de la saveur, des agents conservateurs tels que les esters de l'acide parahydroxybenzoïque, des agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration, plusieurs exemples de préparation des composés actifs de formule (I) selon l'invention, ainsi que des exemples de formulation les contenant.

### EXEMPLES

### EXEMPLE 1 : (2E,4E)-6-[4-(6-tert-butyl-1,1-diméthylindanyl)]-3-méthylhepta-2,4,6-triénoate de méthyle

### (a) (2E,4E)-5-(tri-n-butylstannyl)-3-méthylpenta-2,4-dièn-1-ol

Dans un ballon placé sous argon, on introduit du CuCN (2,01 g ; 22,40 mmol) puis ajoute du THF (60 ml) et refroidit le mélange à -78°C. On ajoute alors goutte à goutte du n-butyl-lithium (1,6 M ; 27,7 ml ; 44,40 mmol) et laisse remonter lentement le mélange à température ambiante. On refroidit à nouveau à -78 °C et ajoute *n*-Bu₃SnH (12 ml ; 44,40 mmol) et agite durant 10 minutes pendant lesquelles l'hydrogène s'évacue. On ajoute alors du (2*E*)-3-méthylpent-2-èn-4-yn-1-ol (1,86 g ; 19,31 mmol) et laisse sous agitation 45 minutes, puis verse le milieu réactionnel dans 200 ml d'une solution de NH₄OH/NH₄Cl (10:90). On extrait à l'éther (3 x 100 ml), sèche sur Na₂SO₄, et évapore sous vide. Après chromatographie sur silice en éluant avec le mélange hexane/acétate d'éthyle/triéthylamine (89/10/1), on obtient 6,28 g (84 %) du composé attendu sous forme d'une huile.
^{**1**}**H RMN** (250,13 MHz, CDCl₃) δ 0,9-1,2 (m, 15H, *n*Bu), 1,3-1,5 (m, 6H, *n*Bu), 1,5-1,7 (m, 6H, nBu), 1,78 (s, 3H, CH₃), 4,30 (t, *J =* 6,2 Hz, 2H, 2H₁), 5,64 (t, *J =* 6,2 Hz, 1H, H₂), 6,25 (d, *J* = 19,3 Hz, ²J_{Sn-H} = 68,0 Hz, 1H, H₅), 6,56 (d, *J* = 19,3 Hz, ³J_{Sn-H} = 63,0 Hz, 1H, H₄) ppm ;
^{**13**}**C RMN** (75,5 MHz, CDCl₃) δ 9,9 (t, ¹J_{Sn-C} = 343,9/328,9 Hz, 3x), 12,3 (q, 3x), 14,1 (q), 27,7 (t, ²J_{Sn-C} = 54,3 Hz, 3x), 29,5 (t, ³J_{Sn-C} = 20,5 Hz, 3x), 59,9 (t), 128,7 (d, ¹J_{Sn-C} = 383,5/356,9 Hz, C₅), 130,2 (d, ²J_{Sn-C} = 139,6 Hz, C₄), 138,1 (s, ³J_{Sn-C} = 65,1 Hz, C₃), 150,4 (d) ppm ;
**MS** (EI⁺) m/e (intensité relative) 386 (M⁺, 0,4), 335 (17), 333 (15), 332 (16), 331 ([M-Bu+1]⁺, 100), 330 (36), 329 (75), 328 (28), 327 (42), 279 (17), 275 (98), 274 (33), 273 (74), 272 (27), 271 (43), 219 (86), 218 (27), 217 (69), 216 (24), 215 (42), 179 (18), 177 (27), 175 (21), 137 (63), 136 (17), 135 (50), 134 (6), 133 (30), 121 (42), 120 (21), 119 (33), 118 (16), 117 (19), 81 (25), 79 (25), 77 (16) ;
**HRMS** (EI⁺) calc. : pour C₁₈H₃₆OSn 386,1782, trouvé : 386,1788 ;
**FTIR** ν 3800-3600 (br, -OH), 2962 (s, C-H), 2928 (s, C-H), 2857 (s), 1567 (m), 1460 (s, C-O), 1380 (w), 1184 (m), 1078 (m), 992 (s) cm⁻¹.

### (b) (2E,4E)-5- (tri-n-butylstannyl)-3-méthylpenta-2,4-dièn-1-al

A une solution refroidie (-10°C) du composé obtenu en **1a** ci-dessus (387 mg ; 1,00 mmol) et de triéthylamine (0,42 ml ; 3,00 mmol) dans du dichlorométhane (3,3 ml), on ajoute le complexe anhydride sulfurique-pyridine (477 mg ; 3,00 mmol). Le mélange est agité -vigoureusement pendant 30 minutes à 0°C, puis est versé dans une solution aqueuse de chlorure de sodium (10 ml, 1:1) et on procède à une extraction avec de l'éther éthylique (2 x 6 ml). Les phases organiques sont lavées avec une solution aqueuse d'acide citrique à 10 % (4 ml) et une solution de chlorure de sodium refroidie (2 x 4 ml). On décante et sèche ensuite sur MgSO₄ et évapore. Le résidu est purifié par HPLC en phase inverse (CH₃CN) pour conduire à 371 mg (96 %) du composé attendu sous forme d'une huile.
^{**1**}**H RMN** (250,13 MHz, CDCl₃) δ 0,8-1,1 (m, 15 H, *n*Bu), 1,2-1,4 (m, 6H, *n*Bu), 1,4-1,6 (m, 6H, *n*Bu), 2,24 (s, 3H, CH₃), 5,90 (d, *J* = 8,2 Hz, 1H, H₂), 6,67 (d, *J* = 19,3 Hz, ³J_{Sn-H} = 57,7 Hz, 1H, H₄), 7,02 (d, *J* = 19,3 Hz, ²J_{Sn-H} = 59,0 Hz, 1H, H₅), 10,14 (d, *J* = 8,2 Hz, 1H, CHO) ppm ;
^{**13**}**C RMN** (75,5 MHz, CDCl₃) δ 10,1 (t, ¹J_{Sn-C} = 347,8/333,2 Hz, 3x), 12,8 (q), 14,0 (q, 3x), 27,6 (t, ²J_{Sn-C} = 55,1 Hz, 3x), 29,4 (t, ³J_{Sn-C} = 21,1 Hz, 3x), 129,5 (d), 142,0 (d, ¹J_{Sn-C} = 336,3 Hz, C₅), 149,1 (d), 154,8 (s), 192,6 (d, CHO) ppm ;
**MS** (EI⁺) *m/e* (intensité relative) 329 ([M-Bu+1]⁺, 85), 328 (31), 327 (62), 326 (24), 325 (36), 273 (44), 272 (15), 271 (33), 269 (20), 17 (71), 216 (25), 215 (58), 213 (35), 137 (29), 135 (25), 95 ([M-SnBu₃]⁺, 100) ;
**HRMS** (EI⁺) calc. : pour C₁₈H₃₄OSn 386,1632, trouvé : 386,1618 ;
**FTIR** (CHCl₃) ν 2966 (s, C-H), 2922 (s, C-H), 2857 (s, C-H), 1668 (s, C=O), 1600 (w, C=C), 1453 (w), 1197 (m), 1113 (m), 991 (m), 870 (m), 687 (m)cm⁻¹.

### (c) (2E,4E) -5- (tri-n-butylstannyl) -3-méthylpenta-2,4-diénoate de méthyle

A une solution refroidie (0°C) de l'aldéhyde obtenu en 1b ci-dessus (0,37 g ; 0,96 mmol) dans le méthanol (4,8 ml), on ajoute un mélange de KCN (0,31 g ; 4,82 mmol) et de MnO₂ (1,67 g ; 19,26 mmol). Le mélange résultant est agité à 0°C durant 90 minutes et filtré à travers de la célite. Après élimination du solvant, le résidu est purifié par chromatographie sur silice en éluant avec le mélange hexane/acétate d'éthyle (95:5) pour conduire à 327 mg (82 %) du composé attendu sous forme d'une huile.
^{**1**}**H RMN** (250,13 MHz, CDCl₃) δ 0,8-0,9 (m, 15 H, *n*Bu), 1,2-1,3 (m, 6H, *n*Bu), 1,4-1,5 (m, 6H, *n*Bu), 2,25 (d, *J* = 1,1 Hz, 3H, CH₃), 3,71 (s, 3H, CO₂CH₃), 5,74 (br s, 1H, H₂), 6,56 (d, *J* = 19,4 Hz, ³J_{Sn-H} = 46,2 Hz, 1H, H₄), 6,80 (d, *J* = 19,4 Hz, ²J_{Sn-H} = 51,3 Hz, 1H, H₅) ppm ;
^{**13**}**C RMN** (75,5 MHz, CDCl₃) δ 10,0 (t, ¹J_{Sn-C} = 346,5/331,1 Hz, 3x), 13,5 (q), 14,0 (q, 3x), 27,6 (t, ²J_{Sn-C} = 54,6 Hz, 3x), 29,4 (t, ³J_{Sn-C} = 20,8 Hz, 3x), 51,4 (q, CO₂CH₃), 118,6 (d), 138,4 (d, ¹J_{Sn-C} = 352,6/337,5 Hz, C₅), 149,4 (d, ⁴J_{Sn-C} = 10, 9 Hz, C₅), 153,3 (s, ³J_{Sn-C} = 62,5 Hz, C₃), 168,0 (s, CO₂CH₃) ppm,
**MS** (EI⁺) *m/e* (intensité relative) 359 ([M-Bu+1]⁺, 75), 358 (28), 357 (56), 356 (22), 355 (32), 303 (81), 302 (28), 301 (60), 300 (23), 299 (35), 251 (18), 250 (15), 247 (100), 246 (32), 245 (77), 244 (28), 243 (46), 151 (62), 150 (16), 149 (46), 148 (14) ;
**HRMS** (EI⁺) calc. : pour C₁₉H₃₆O₂Sn 416,1737, trouvé : 416,1726 ;
**FTIR** (CHCl₃) ν 2955 (m, C-H), 2926 (m, C-H), 2852 (w, C-H), 1717 ( s, C=O), 1615 (w, C=C), 1558 (w),1435 (w), 1232 (m), 1152 (s) cm⁻¹.

### (d) 6-tert-butyl-4-éthynyl-1,1-diméthylindane

On prépare du lithium diisopropylamide (LDA) en ajoutant du butyllithium(1,6 M dans l'hexane ; 16,11 ml ; 25,78 mmol) à une solution de diisopropylamine (3,6 ml ; 25,78 mmol) dans le THF (24 ml) à 0°C. Le mélange est agité 30 minutes à cette température, puis est refroidi a -78° C. On ajoute alors lentement du 4-acétyl-6*-tert*-butyl-1,1-diméthylindane (6,0 g ; 24,55 mmol). Après agitation pendant 1 heure à -78°C, on ajoute du chlorophosphate de diéthyle (3,72 ml ; 25,78 mmol) et laisse le milieu réactionnel revenir à température ambiante sur une période de 2-3 heures (mélange A). On prépare séparément du LDA à partir de diisopropylamine (7,74 ml ; 55,24 mmol), et du butyllithium(1,6 M dans l'hexane ; 34,52 ml ; 55,24 mmol) dans du THF (95 ml) à 0°C. Après agitation durant 30 minutes et refroidissement à -78°C, on y ajoute le mélange A ci-dessus, préalablement refroidi à -78°C. On laisse revenir sous agitation le mélange réactionnel résultant sur une période de 2-3 heures puis traite avec de l'eau à 0°C. On extrait avec de l'hexane (3 x 50 ml), lave avec HCl 1N (100 ml) glacé et une solution saturée de NaHCO₃ (100 ml), puis sèche sur MgSO₄ et évapore. Le résidu est purifié par chromatographie sur silice en éluant avec de l'hexane pour conduire à 4,89 g (88 %) du composé attendu sous forme d'un solide blanc.
^{**1**}**H RMN** (250,13 MHz, CDCl₃) δ 1,26 (d, *J* = 0,9 Hz, 6H, 2x CH₃), 1,32 (d, *J* = 1,0 Hz, 9H, *t*Bu), 1,95 (dt, *J* = 7,2, 0,9 Hz, 2H, 2H₂), 2,95 (t, *J* = 7,2 Hz, 2H, 2H₃), 3,17 (s, 1H, H_{2'}), 7,15 (s, 1H, Ar-H), 7,33 (br s, 1H, Ar-H) ppm ;
^{**13**}**C RMN** (62,89 MHz, CDCl₃) δ 28,6 (q), 29,1 (t), 31,4 (q), 34,6 (s), 41,0 (t), 44,5 (s) 78,9 (s), 83,0 (d), 117,6 (s), 119,8 (d), 127,1 (d), 143,1 (s), 149,9 (s), 152,6 (s) ppm ;
**MS** (EI⁺) *m/e* (intensité relative) 226 (M⁺, 22), 212 (18), 211 ([M-CH₃]⁺, 100), 165 (8), 155 (16), 153 (8), 152 (7), 58 (37), 57 (10) ;
**HRMS** (EI⁺) calc. : pour C₁₇H₂₂ 226,1723, trouvé : 226,1721 ; **FTIR** (CDCl₃) ν 3290 (m, ≡C-H), 2954 (s, C-H), 2864 (m, C-H), 2103 (w, C≡C), 1580 (w), 1463 (m), 1362 (m), 1316 (w), 1253 (w), 878 (m), 650 (m) cm⁻¹.

### (e) 6-tert-butyl-4-(1-iodoéthèn-1-yl) -1,1-diméthylindane

On ajoute par fractions une solution de Me₃SiCl (1,04 ml, 8,30 mmol) dans l'eau (0,07 ml) à une solution d'iodure de sodium (1,24 g, 8,30 mmol) dans l'acétonitrile (23 ml). Après 10 minutes, l'alcyne obtenu en 1d ci-dessus (1,50 g, 6,64 mmol) est alors ajouté à cette première solution et le mélange est agité pendant 3 heures. On ajoute ensuite 10 ml d'eau et extrait à l'éther (3 x 50 ml), puis sèche sur MgSO₄. Après évaporation, le résidu est purifié par chromatographie flash (SiO₂, hexane) pour conduire à 2,29 g, (97 %) du composé attendu sous forme d'une huile.
^{**1**}**H RMN** (300,13 MHz, CDCl₃) δ 1,27 (s, 6H, 2 x CH₃), 1,34 (s, 9H, *t*Bu), 1,93 (t, *J* = 7,2 Hz, 2H, H₃), 2,88 (t, *J =* 7,2 Hz, 2H, H₂), 6,15 (d, *J* = 1,2 Hz, 1H, H₂,), 6,18 (d, *J* = 1,2 Hz, 1H, H₂,), 7,10 (br s, 1H, Ar-H), 7,18 (br s, 1H, Ar-H) ppm ;
^{**13**}**C RMN** (75,47 MHz, CDCl₃) δ 29,1 (q, 2x), 29,7 (t), 32,0 (q, 3x), 35,2 (s), 41,9 (t), 44,5 (s), 105,8, 119,7 (d), 124,6 (d), 129,5 (t), 137,2 (s), 139,3 (s), 150,3 (s), 153,3 (s) ppm ;
**MS** (EI⁺) *m/e* (intensité relative) 354 (M⁺, 3), 243 (2), 227 ([M-I]⁺, 100), 212 (6), 197 (6), 165 (7), 155 (10), 153 (8), 152 (7), 141 (8), 129 (6), 128 (8), 127 (4), 115 (8), 57 (32) ;
**HRMS** (EI⁺) calc. : pour C₁₇H₂₃I 354,0845, trouvé : 354,0844 ;
**FTIR** (CHCl₃) v 2953 (s, C-H), 2862 (s, C-H), 1598 (m, C=C), 1476 (m), 1460 (m), 1361 (m), 1252 (w), 1202 (w), 896 (m), 878 (m), 656 (w), 643 (w) cm⁻¹.

### (f) (2E,4E)-6-[4-(6-tert-butyl-1,1-diméthylindanyl)]-3-méthylhepta-2,4,6-triénoate de méthyle

On ajoute de la triphénylarsine (AsPh₃) (1,4 mg ; 0,005 mmol) et du Pd₂(dba)₃ (complexe tris(dibenzylidène acetone)palladium) (4,1 mg ; 0,005 mmol) à une solution du dérivé iodé obtenu en 1e ci-dessus (53 mg 0,150 mmol) dans de la N-méthyl-morpholine (NMP) anhydre (2,1 ml). Après 10 minutes, une solution de stannate obtenu en 1c ci-dessus (68 mg ; 0,165 mmol) dans de la NMP (1 ml) est ajoutée, et la solution est agitée à 80°C pendant 90 minutes. Après addition de 2 ml d'une solution aqueuse saturée de KF, le mélange est extrait avec de l'éther (3 x 5 ml). Les extraits organiques sont lavés avec H₂O (3 x 5 ml), puis on sèche sur MgSO₄ et évapore. Le résidu est alors purifié par chromatographie sur silice en éluant avec le mélange hexane/acétate d'éthyle (95:5) pour conduire à 40 mg (76 %) d'un solide jaune.
^{**1**}**H RMN** (250,13 MHz, CDCl₃) δ 1,29 (s, 6H, 2 x CH₃), 1,35 (s, 9H, *t*Bu), 1,88 (t, *J =* 7,1 Hz, 2H, 2H₂,), 2,36 (d, *J* = 1,0 Hz, 3H, CH₃), 2,67 (t, *J* = 7,1 Hz, 2H, 2H₃,), 3,69 (s, 3H, CO₂CH₃), 5,27 (d, *J* = 1,7 Hz, 1H, H₂), 5,53 (s, 1H, H₇), 5,67 (s, 1H, H₇), 5,93 (d, *J* = 15,8 Hz, 1H, H₅), 6,83 (d, *J* = 15,8 Hz, 1H, H₄), 7,01 (d, *J* = 1,7 Hz, 1H, Ar-H), 7,15 (d, *J* = 1,7 Hz, 1H, Ar-H) ppm ;
^{**13**}**C RMN** (62,89 MHz, CDCl₃) δ 13,7 (q), 28,6 (q, 2x), 28,8 (t), 31,6 (q, *t*Bu), 34,6 (s), 41,5 (t), 44,1 (s), 50,9 (q), 118,1 (d), 119,4 (d), 120,6 (t), 124,0 (d), 134,9 (d), 135,2 (s), 135,9 (d), 137,9 (s), 147,8 (s), 149,8 (s), 152,5 (s), 152,6 (s), 167,4 (s) ppm ;
**MS** (EI⁺) *m/e* (intensité relative) 352 (M⁺, 100), 337 ([M-CH₃]⁺, 23), 277 (46), 249 (45), 221 (32), 181 (45), 165 (33), 58 (57), 57 (78) ;
**HRMS** (EI⁺) calc. : pour C₂₄H₃₂O₂ 352,2404, trouvé : 352,2398 ;
**FTIR** (CHCl₃) **ν** 2954 (s, C-H), 2871 (m, C-H), 1718 (s, C=O), 1624 (m, C=C), 1451 (m), 1373 (w), 1214 (m), 1155 (s), 1048 (m), 879 (w), 751 (m).

### EXEMPLE 2 : Acide (2E,4E)-6-[4-(6-tert-butyl-1,1-diméthylindanyl)]-3-méthylhepta-2,4,6-triénoïque

Le dérivé stannique obtenu à l'exemple 1 (1c) (100 mg ; 0,241 mmol) dans 0,5 ml d'éther est ajouté goutte à goutte sous agitation à une solution de KOSiMe₃ (90 %, 34 mg ; 0,241 mmol) dans l'éther (1,5 ml) et le milieu réactionnel est agité pendant 7 heures à 50°C (solution A).
De la triphénylarsine (AsPh₃) (2,0 mg ; 0,007 mmol) et du complexe Pd₂(dba)₃ (6,0 mg ; 0,007 mmol) sont ajoutés à une solution de l'iodure obtenu à l'exemple 1 (1e) (77 mg ; 0,22 mmol) dans de la NMP anhydre (3 ml). Après 10 minutes d'agitation, on ajoute alors la solution A ci-dessus et agite le milieu réactionnel pendant 8 heures à température ambiante. Après avoir ajouté 3 ml d'une solution saturée de fluorure de potassium, on extrait à l'éther (3 x 7 ml) et les phases organiques rassemblées sont séchées sur MgSO₄ puis évaporées. Le résidu est purifié par chromatographie sur silice en éluant à l'aide du mélange hexane/acétate d'éthyle (90:10) pour conduire à 34 mg (46 %) du produit attendu sous forme d'un solide jaune.
^{**1**}**H RMN** (250,13 MHz, CDCl₃) δ 1,23 (s, 6H, 2 x CH₃), 1,29 (s, 9H, *t*Bu), 1,83 (t, *J* = 6,9 Hz, 2H, 2H₂.), 2,28 (s, 3H, CH₃), 2,62 (t, *J* = 6,9 Hz, 2H, 2H₃,), 5,22 (s, 1H, H₁), 5,48 (s, 1H, H₇), 5,62 (s, 1H, H₇), 5,89 (d, *J* = 15,8 Hz, 1H, H₄), 6,78 (d, *J* = 15,3 Hz, 1H, H₅), 6,95 (s, 1 H, Ar-H), 7,70 (s, 1H, Ar-H) ppm ;
^{**13**}**C RMN** (62,89 MHz, CDCl₃) δ 13,7 (q), 28,5 (q, 2x), 28,6 (t), 31,5 (q, *t*Bu), 34,5 (s), 41,4 (t), 44,0 (s), 118,0 (d), 119,3 (d), 120,5 (t), 124,0 (d), 134,9 (d), 135,1 (s), 135,9 (d), 137,8 (s), 147,8 (s), 149,7 (s), 152,6 (s), 152,7 (s), 167,7 (s), 171,8 (s) ppm ;
**MS** (EI⁺) *m/e* (intensité relative) 338 (M⁺, 100), 323 (52), 249 (36), 234 (54), 233 (52), 181 (33), 179 (25), 165 (32), 131 (20), 128 (22), 103 (22), 91 (18), 77 (29), 57 (67) ;
**HRMS** (EI⁺) calc. : pour C₂₃H₃₀O₂ 338,2246, trouvé : 338,2242 ;
**FTIR** (CHCl₃) **ν** 2953 (s, C-H), 2860 (m, C-H), 1683 (m, C=O), 1603 (s, C=C), 1451 (w), 1359 (w), 1248 (m), 1186 (m), 967 (w), 881 (w) ; **UV** (CH₃OH)λₘₐₓ (ε), 286 (15700) nm.

### EXEMPLE 3 : (2E,4E)-6-[4-(6-tert-butyl-1,1-diméthylindanyl)]hepta-2,4,6-triènoate de méthyle

### (a) (2E,4E)-5-(tri-n-butylstannyl)penta-2,4-dièn-1-ol

Dans un ballon placé sous argon, on introduit du CuCN (0,63 g ; 7,06 mmol), puis ajoute du THF (18 ml) et refroidit le mélange à -78°C. On ajoute alors goutte à goutte du n-butyl-lithium (1,6 M; 8,8 ml; 14,01 mmol) et laisse remonter lentement le mélange à température ambiante. On refroidit à nouveau à -78°C et ajoute *n*-Bu₃SnH (3,8 ml; 14,01 mmol) et agite durant 10 minutes pendant lesquelles l'hydrogène s'évacue. On ajoute alors le (2E)-pent-2-èn-4-yn-1-ol (0,50 g ; 6,09 mmol) et laisse sous agitation 45 minutes avant de verser ce milieu réactionnel dans 200 ml d'une solution de NH₄OH/NH₄Cl (10:90). On extrait à l'éther (3 x 100 ml), sèche sur Na₂SO₄, et évapore sous vide pour obtenir, après chromatographie sur silice en éluant avec le mélange hexane/acétate d'éthyle/triéthylamine, (89:10:1), 1,52 g du produit attendu (67 %) sous forme d'une huile.
^{**1**}**H RMN** (250,13 MHz, CDCl₃) δ 0,8-1,1 (m, 15 H, nBu), 1,2-1,4 (m, 6H, nBu), 1,4-1,6 (m, 6H, *n*Bu), 4,20 (br s, 2H, 2H₁), 5,79 (dt, *J* = 15,6, 5,8 Hz, 1H, H₂), 6,21 (dd, *J* = 15,6, 10,0 Hz, 1H, H₃), 6,26 (d, *J* = 18,5 Hz, ²J_{Sn-H} = 67,2 Hz, 1H, H₅), 6,54 (dd, J = 18,5, 10,0 Hz, ³J_{Sn-H} = 59,2 Hz, 1H, H₄) ppm ;
^{**13**}**C RMN** (62,89 MHz, CDCl₃) δ 9,6 (t, ¹J_{Sn-C} = 344,2/330,6 Hz, 3x), 13,8 (q, 3x), 27,4 (t, ²J_{Sn-C} = 53,7 Hz, 3x), 29,3 (t, ³J_{Sn-c}= 20,3 Hz, 3x), 63,5 (t, C₁), 131,0 (d), 134,9 (d, ¹J_{Sn-C} = 315,4 Hz, C₅), 135,4 (d), 146,2 (d) ppm ;
**MS** (EI⁺) *m/e* (intensité relative) 317 ([M-Bu+1]⁺, 100), 315 (75), 313 (42), 261 (48), 259 (37), 257 (21), 251 (31), 249 (23), 247 (16), 205(56), 203 (47), 201 (29), 137 (64), 135 (49), 133 (29), 121 (36), 119 (28), 117 (16) ;
**HRMS** (EI⁺) calc. : pour C₁₇C₃₅OSn 373,1704, trouvé : 373, 1693 ;
**FTIR** (CHCl₃) v 3600-3200 (br, -OH), 2960 (s, C-H), 2925 (s, C-H), 2864 (s, C-H), 1579 (w, C=C), 1456 (m, C-O), 1006 (m) cm⁻¹.

### (b) (2E,4E)-5- (tri-n-butylstannyl)penta-2,4-diènal

Selon le même mode opératoire que celui décrit pour la préparation du composé de l'exemple 1(1b), on traite une solution du composé obtenu en **3a** ci-dessus (500 mg ; 1,34 mmol) et de triéthylamine (0,56 ml; 4,02 mmol) dans CH₂Cl₂ (4,5 ml) avec du complexe anhydride sulfurique-pyridine (640 mg ; 4,02 mmol). On obtient après le même traitement 348 mg (70 %) de composé attendu sous forme d'une huile.
^{**1**}**H RMN** (250,13 MHz, CDCl₃) δ 0,8-1,2 (m, 15H, nBu), 1,2-1,4 (m, 6H, *n*Bu), 1,4-1,6 (m, 6H, *n*Bu), 6,06 (dd, *J* = 15,0, 8,0 Hz, 1H, H₂), 6,79 (dd, *J* = 18,8, 10,2 Hz, ³J_{Sn-H} = 53,3 Hz, 1H, H₄), 7,00 (dd, *J* = 15,3, 10,2 Hz, H₃), 7,10 (d, *J* = 18,8 Hz, ²J_{Sn-H} = 60,7 Hz, 1H, H₅), 9,56 (d, *J* = 8,0 Hz, 1H, CHO) ppm ;
^{**13**}**C RMN** (62,89 MHz, CDCl₃) δ 9,6 (t, ¹J_{Sn-C} = 349,2/330,0 Hz, 3x), 13,6 (q, 3x), 27,2 (t, ²J_{Sn-C} = 54,8 Hz, 3x), 29,0 (t, ³J_{Sn-C} = 20,3 Hz, 3x), 130,1 (d), 144,3 (d), 151,5 (d, ¹J_{Sn-C} = 325,5/310,3 Hz, C₅), 153,6 (d, ²J_{Sn-C} = 67,8 Hz, C₄), 194,5 (d, C₁) ppm ;
**MS** (EI⁺) *m/e* (intensité relative) 315 ([M-Bu+1]⁺, 88), 314 (31), 313 (64), 312 (24), 311 (36), 259 (66), 258 (23), 257 (49), 256 (19), 255 (29), 203 ([M-Bu₃]⁺, 100), 202 (38), 201 (84), 200 (33), 199 (52), 173 (26), 171 (19), 169 (10), 121 (47), 119 (39), 117 (23), 81 ([M-SnBu₃]⁺, 76) ;
**HRMS** (EI⁺) calc. : pour C₁₇H₃₂OSn 372,1475, trouvé : 372,1483.

### (c) (2E,4E)-5-(tri-n-butylstannyl)penta-2,4-diènoate de méthyle

Selon le même mode opératoire que celui décrit pour la préparation du composé de l'exemple 1 (1c), on traite une solution d'aldéhyde obtenue en 3b ci-dessus (0,35 g ; 0,94 mmol) dans le méthanol (4,7 ml) en ajoutant un mélange de KCN (0,32 g ; 4,93 mmol) et de MnO₂ (1,63 g ; 18,76 mmol). Après le même traitement, on isole 370 mg (98 %) du composé attendu sous forme d'une huile.
^{**1**}**H RMN** (250,13 MHz, CDCl₃) δ 0,8-1,0 (m, 15 H, *n*Bu), 1,2-1,4 (m, 6H, *n*Bu), 1,4-1,6 (m, 6H, *n*Bu), 3,74 (s, 3H, CO₂CH₃), 5,80 (d, *J* = 15,4 Hz, 1H, H₂), 6,64 (dd, *J* = 18,7, 9,9 Hz, ³J_{Sn-H} = 54,3 Hz, 1H, H₄), 6,82 (d, J = 18,7 Hz, ²J_{Sn-H} = 63,2 Hz, 1H, H₅), 7,19 (dd, *J* = 15,4, 9,9 Hz, ⁴J_{Sn-H} = 5,2 Hz, 1H, H₃) ppm ;
^{**13**}**C RMN** (62,89 MHz, CDCl₃) δ 9,6 (t, ¹J_{Sn-C} = 347,8/335,1 Hz, 3x), 13,6 (q, 3x), 27,2 (t, ²J_{Sn-C} = 54,4 Hz, 3x), 29,0 (t, ³J_{Sn-C} = 20,4 Hz, 3x), 51,4 (q, CO₂CH₃), 119,4 (d), 144,2 (d), 146,7 (d, ²J_{Sn-C} = 69,9 Hz, C₄), 147,5 (d, ¹J_{Sn-C} = 340,8/327,2 Hz, C₅), 167,9 (s, CO₂CH₃) ppm.
**MS** (EI⁺) *m/e* (intensité relative) 345 ([M-Bu+1]⁺, 100), 344 (36), 343 (73), 342 (28), 341 (41), 289 (53), 288 (18), 287 (40), 286 (15), 285 (23), 233 (76), 232 (24), 231 (61), 230 (21), 229 (36), 177 (13), 175 (10), 151 (41), 149 (32), 147 (21), 121 (18) ;
**HRMS** (EI⁺) calc. : pour C₁₈H₃₄O₂Sn 402,158, trouvé : 402, 1581 ;
**FTIR** (CHCl₃) **ν** 2956 (m, C-H), 2925 (m, C-H), 2853 (m, C-H), 1722 (s, C=O), 1626 (w, C=C), 1560 (w), 1435 (w), 1458 (w), 1439 (w), 1273 (m), 1213 (m), 1154 (m), 1101 (w), 1010 (w), 870 (w) cm⁻¹.

### (d) (2E,4E)-6-[4-(6-tert-butyl-1,1-diméthylindanyl)]hepta-2,4,6-triènoate de méthyle

De la triphénylarsine (AsPh₃) (1,4 mg ; 0,005 mmol) et du complexe Pd₂(dba)₃ (4,1 mg ; 0,005 mmol) sont ajoutés à une solution du dérivé iodé obtenu à l'exemple 1 (1e) (53 mg ; 0,15 mmol) dans de la NMP anhydre (2,1 ml). Après 10 minutes d'agitation on ajoute une solution du dérivé obtenu en 3c ci-dessus (66 mg; 0,165 mmol) dans de la NMP (1 ml) puis agite cette solution à 60°C pendant 1 heure. Après avoir ajouté 2 ml d'une solution saturée de fluorure de potassium, le milieu est extrait à l'éther (3 x 5 ml) et les phases organiques rassemblées sont lavées à l'eau (3 x 5 ml), séchées sur MgSO₄ et évaporées. Le résidu est purifié par chromatographie sur *si*lice en éluant à l'aide du mélange hexane/acétate d'éthyle, (95:5) pour conduire à 42 mg (83 %) du composé attendu sous forme d'un solide jaune.
^{**1**}**H RMN** (250,13 MHz, CDCl₃) δ 1,28 (s, 6H, 2 x CH₃), 1,33 (s, 9H, *t*Bu), 1,87 (t, *J* = 7,2 Hz, 2H, 2H₂,), 2,66 (t, *J* = 7,2 Hz, 2H, 2H₃,), 3,73 (s, 3H, CO₂CH₃), 5,30 (s, 1H, H₇), 5,53 (s, 1H, H₇), 5,79 (d, *J* = 15,3 Hz, 1H, H₅), 5,99 (dd, *J* = 15,1, 11,2 Hz, 1H, H₄), 6,77 (d, *J* = 15,3 Hz, 1H, H₂), 7,00 (d, *J* = 1,4 Hz, 1H, Ar-H), 7,14 (d, *J* = 1,4 Hz, 1H, Ar-H), 7,37 (dd, *J* = 15,2, 11,2 Hz, 1H, H₃) ppm ;
^{**13**}**C RMN** (62,89 MHz, CDCl₃) δ 28,6 (q, 2x), 28,7 (t), 31,6 (q, *t*Bu), 34,6 (s), 41,5 (t), 44,1 (s), 51,4 (q), 118,2 (d), 121,0 (d), 121,5 (t), 124,0 (d), 129,3 (d), 134,9 (s), 137,9 (s), 142,1 (d), 144,7 (d), 147,6 (s), 149,8 (s), 152,7 (s), 167,5 (s) ppm ;
**MS** (EI⁺) *m/e* (intensité relative) 338 (M⁺, 100), 323 ([M-CH₃]⁺, 100), 291 (16), 249 (37), 237 (35), 235 (38), 207 (41), 193 (23), 191 (25), 179 (33), 178 (29), 167 (26), 165 (47), 152 (36), 57 (67) ;
**HRMS** (EI⁺) calc. : pour C₂₃H₃₀O₂ 338,2246 trouvé : 338,2239 ;
**FTIR** (CHCl₃) v 2950 (s, C-H), 2900 (m, C-H), 1716 (s, C=O), 1622 (s, C=C), 1449 (m), 1245 (s), 1147 (s), 1008 (m), 893 (w) cm⁻¹ ;
**UV** (CH₃OH) λₘₐₓ (ε), 206 (11600) nm,

### EXEMPLE 4 : Acide (2E,4E)-6-[4-(6-tert-butyl-1,1-diméthylindanyl)]hepta-2,4,6-triènoïque

Une solution de l'ester obtenu à l'exemple 3 ci-dessus (24 mg ; 0,07 mmol) dans 0,2 ml d'éther est ajoutée goutte à goutte à une solution de KOSiMe₃ (90 %, 10 mg ; 0,07 mmol) dans de l'éther (0,5 ml) et le milieu réactionnel est agité 12 heures à 40°C. Après avoir ajouté 2 ml d'eau et agité 1 heure à température ambiante, le pH de la solution est ajusté à 4-5 par addition d'HCl 0,1N. La phase aqueuse est extraite à l'éther (5 x 5 ml) et les phases organiques rassemblées sont séchées sur MgSO₄, filtrées et concentrées sous vide. La purification par chromatographie préparative sur couche mince de silice à l'aide de l'éluant hexane/acétate d'éthyle (90:10) conduit à 12 mg (52 %) de l'acide attendu sous forme d'un solide jaune et à 4mg (17 %) de produit de départ 3.
^{**1**}**H RMN** (250,13 MHz, CDCl₃) δ 1,28 (s, 6H, 2 x CH₃), 1,34 (s, 9H, *t*Bu), 1,88 (t, *J* = 7,1 Hz, 2H, 2H₂'), 2,67 (t, *J* = 7,1 Hz, 2H, 2H₃'), 5,34 (s, 1H, H7), 5,57 (s, 1H, H₇), 5,78 (d, *J* = 15,3 Hz, 1H, H₅), 6,01 (dd, *J* = 15,3, 11,4 Hz, 1H, H₄), 6,81 (d, *J* = 15,3 Hz, 1H, H₂), 7,00 (s, 1H, Ar-H), 7,16 (s, 1H, Ar-H), 7,45 (dd, *J* = 15,3, 11,4 Hz, 1H, H₃) ppm ;
^{**13**}**C RMN** (62,89 MHz, CDCl₃) δ 28,6 (q, 2x), 28,7 (t), 31,6 (q, *t*Bu), 34,6 (s), 41,5 (t), 44,1 (s), 118,2 (d), 120,4 (d), 122,2 (t), 124,0 (d), 129,1 (d), 134,7, (s), 137,9 (s), 143,2 (d), 146,8 (d), 147,6 (s), 149,9 (s), 152,7 (s), 171,9 (s) ppm ;
**MS** (EI⁺) *m/e* (intensité relative) 324 (M⁺, 8), 271 (38), 269 (100), 268 (34), 267 (71), 265 (35), 213 (32), 211 (23), 177 (25), 155 (39), 153 (29), 58 (30), 57 (24) ;
**HRMS** (EI⁺) calc. : pour C₂₂H₂₈O₂ 324,2089, trouvé : 324,2091 ;
**FTIR** (CHCl₃) ν 2955 (s, C-H), 2863 (m, C-H), 1687 (s, C=O), 1619 (s, C=C), 1459 (m), 1416 (m), 1363 (m), 1308 (m), 1272 (m), 1252 (m), 1146 (w), 1001 (m), 882 (m) cm⁻¹.
**UV** (CH₃OH)λₘₐₓ (ε) 204 (44646), 284 (34921) nm.

### EXEMPLE 5 : (2E,4E,6E)-7-[4-(6-tert-butyl-1,1-diméthylindanyl)]-3, 7-diméthylhepta-2,4,6-triènoate de méthyle

### (a) 6-tert-butyl-4-([E]-2-iode-1-méthyléthèn-1-yl)-1,1-diméthylindane

A une suspension de Cp₂ZrCl₂ (complexe bis(cyclopentadiènyl)zirconium dichlorure) (117 mg ; 0,4 mmol) dans CH₂Cl₂ (5,3 ml), on ajoute du AlMe₃ (0,4 ml; 4,12 mmol) à 0°C. Le mélange est agité à cette température pendant 10 minutes puis est refroidi à -23°C. On ajoute alors de l'eau (0,04 ml; 2,06 mmol) sous forte agitation et agite 10 minutes supplémentaires, puis ajoute à -23°C, l'alcyne obtenu à l'exemple 1 (1d) (300 mg ; 1,33 mmol) dans du dichlorométhane (2,10 ml). Après avoir agité 30 minutes à cette température, une solution d'iode (676 mg; 2,66 mmol) dans le THF (5,5 ml) est ajoutée goutte à goutte à -50°C. Le mélange résultant est alors agité 5 heures à température ambiante, puis la réaction est stoppée par l'addition d'un mélange THF/H₂O (1:1). On extrait alors avec de l'hexane (3 x 15 ml) et lave avec une solution de Na₂S₂O₃ (2 x 25 ml) dans de l'eau (30 ml). Les phases organiques rassemblées sont séchées sur Na₂SO₄ et concentrées. La chromatographie sur silice en éluant avec de l'hexane conduit à 439 mg du composé attendu (90 %) sous forme d'un solide blanc.
^{**1**}**H RMN** (250,13 MHz, CDCl₃) δ 1,28 (s, 6H, 2 x CH₃), 1,34 (s, 9H, *t*Bu), 1,92 (t, *J* = 7,1 Hz, 2H, 2H₂), 2,22 ( d, *J* = 1,1 Hz, 3H, CH₃), 2,82 (t, *J* = 7,1 Hz, 2H, 2H₃), 6,25 (br s, 1H, H₂,), 7,03 (d, *J* = 1,7 Hz, 1H, Ar-H), 7,13 (d, *J* = 1,7 Hz, 1H, Ar-H) ppm ;
^{**13**}**C RMN** (62,89 MHz, CDCl₃) δ 25,4 (q), 28,6 (q, 2x), 29,2 (t), 31,6 (q, *t*Bu), 34,7 (s), 41,6 (t), 44,0 (s), 79,5 (d), 118 (d), 122,4 (d), 136,7 (s), 138,4 (s), 148,6 (s), 150,1 (s), 153,1 (s) ppm ;
**MS** (EI⁺) *m/e* (intensité relative) 368 (M⁺, 44), 354 (21), 353 ([M-CH₃]⁺, 100), 241 (14), 211 (15), 170 (13), 155 (9), 128 (6) ;
**HRMS** (EI⁺) calc. : pour C₁₈H₂₅I 368,1002, trouvé 368,0997 ;
**FTIR** (CHCl₃) **ν** 2953 (s, C-H), 2863 (m, C-H), 1460 (m), 1363 (w), 1200 (w), 878 (w), 789 (w) cm⁻¹.

### (b) (2E,4E,6E)-7-[4-(6-tert-butyl-1,1-diméthylindanyl)]-3,7-diméthylhepta-2,4,6-triènoate de méthyle

De la triphénylarsine (AsPh₃) (1,9 mg ; 0,006 mmol) et du complexe Pd₂(dba)₃ (5,5 mg ; 0,006 mmol) sont ajoutés à une solution de l'iodure obtenu ci-dessus en **5a** (75 mg ; 0,204 mmol) dans de la NMP anhydre (3 ml). Après 10 minutes, une solution de dérivé stannique obtenu à l'exemple 1 (**1c)** (93 mg ; 0,224 mmol) dans de la NMP (1,5 ml) est ajoutée et la solution est agitée à température ambiante pendant 12 heures. Après addition de 4 ml d'une solution saturée de fluorure de potassium, on extrait le mélange à l'éther (3 x 5 ml). Les phases organiques sont rassemblées, lavées à l'eau (3 x 5 ml.), séchées sur MgSO₄ et évaporées. Le résidu est purifié par chromatographie flash sur silice en éluant à l'aide du mélange hexane/acétate d'éthyle (95/5) pour conduire à 67 mg (90 %) du produit attendu sous forme d'un solide jaune, de point de fusion : 118°C.
^{**1**}**H RMN** (250,13 MHz, CDCl₃) δ 1,30 (s, 6H, 2 x CH₃), 1,36 (s, 9H, *t*Bu), 1,93 (t, J = 7,1 Hz, 2H, 2H₂,), 2,25 (s, 3H, CH₃), 2,42 (s, 3H, CH₃), 2,90 (t, *J* = 7,1 Hz, 2H, 2H₃,), 3,74 (s, 3H, CO₂CH₃), 5,83 (s, 1H, H₂), 6,28 (d, *J* = 11,2 Hz, 1H, H₆), 6,33 (d, *J* = 15,2 Hz, 1H, H₄), 7,04 (dd, *J* = 15,2, 11,2 Hz, 1H, H₅), 7,13 (s, 2H, 2 x Ar-H) ppm ;
^{**13**}**C RMN** (62,89 MHz, CDCl₃) δ 13,8 (q), 17,9 (q), 28,5 (q, 2x), 29,6 (t), 31,6 (q, *t*Bu), 34,7 (s), 41,6 (t), 43,8 (s), 50,9 (q), 118,1 (d), 118,3 (d), 122,2 (d), 128,6 (d), 131,1 (d), 135,3 (d), 137,0 (s), 140,2 (s), 142,3 (s), 149,9 (s), 153,0 (s), 153,1 (s), 167,6 (s) ppm ;
**MS** (EI⁺) *m/e* (intensité relative) 366 (M⁺, 31), 351 (8), 309 (38), 291 (14), 277 (22), 253 (16), 252 (34), 251 (36), 249 (49), 237 (25), 235 (26), 229 (28), 228 (23), 213 (17), 197 (59), 195 (29), 193 (21), 181 (28), 179 (18), 165 (26), 155 (24), 83 (16), 57 (*t*Bu⁺, 100) ;
**FTIR** (CHCl₃) v 2952 (s, C-H), 2865 (m, C-H), 1711 (s, C=O), 1600 (s, C=C), 1444 (m), 1361 (w), 1240 (m), 1155 (s), 963 (w), 879 (w), 754 (m) cm⁻¹ ;
**UV** (CH₃OH)_{λmax} (ε) 334 (21100) nm.

### EXEMPLE 6 : Acide (2E,4E,6E)-7-[4-(6-tert-butyl-1,1-diméthylindanyl)]-3, 7-diméthylhepta-2,4, 6-triènoïque

Une solution de l'ester obtenu à l'exemple **5** ci-dessus (45 mg ; 0,123 mmol) dans 0,3 ml d'éther est ajoutée, goutte à goutte, à une solution sous agitation de KOSiMe₃ (90 %, 17 mg ; 0,123 mmol) dans de l'éther (0,75 ml) et le milieu réactionnel est agité pendant 12h à 40°C. Après addition de 2 ml d'eau et agitation pendant 1 heure à température ambiante, le pH de la solution est ajusté à 4-5 par addition d'HCl 0,1N. La phase aqueuse est alors extraite à l'éther et les phases organiques rassemblées sont séchées sur MgSO₄, filtrées et concentrées sous vide. Le résidu est purifié par chromatographie flash sur silice, à l'aide de l'éluant hexane/acétate d'éthyle (80/20) pour conduire à 37 mg (85 %) de l'acide attendu sous forme d'un solide jaune, de point de fusion : 185-190°C.
^{**1**}**H RMN** (250,13 MHz, CDCl₃) δ 1,32 (s, 6H, 2 x CH₃), 1,38 (s, 9H, *t*Bu), 1,94 (t, *J* = 7,0 Hz, 2H, 2H_{2'}), 2,28 (s, 3H, CH₃), 2,44 (s, 3H, CH₃), 2,92 (t, *J* = 7,0 Hz, 2H, 2H₃,), 5,87 (s, 1H, H₂), 6,31 (d, *J* = 11,7 Hz, 1H, H₆), 6,38 (d, *J* = 15,4 Hz, 1H, H₄), 7,10 (dd, *J* = 15,4, 11,7 Hz, 1H, H₅), 7,15 (s, 2H, 2 x Ar-H) ppm ;
^{**13**}**C RMN** (62,89 MHz, CDCl₃) δ 14,0 (q), 18,0 (q), 28,5 (q, 2x), 29,7 (t), 31,6 (q, *t*Bu), 34,7 (s), 41,7 (t), 43,8 (s), 18,2 (d, 2x), 122,3 (d), 128,6 (d), 131,8 (d), 135,2 (d), 137,0 (s), 140,1 (s), 143,0 (s), 149,9 (s), 153,2 (s), 155,3 (s), 172,7 (s) ppm ;
**FTIR** (CHCl₃) ν 3033 (w, C-H), 2959 (s, C-H), 2863 (m, C-H), 1715 (s, C=O), 1610 (s, C=C), 1568 (w), 1448 (m), 1308 (m), 1254 (m), 1140 (m), 1004 (m), 869 (w) cm⁻¹.

### EXEMPLE 7 : (2E,4E,6E)-7-[4-(6-tert-butyl-1,1-diméthylindanyl)]-7-méthylhepta-2,4,6-triènoate de méthyle

On ajoute à une solution de l'iodure obtenu à l'exemple 5 (5a) (75 mg ; 0,204 mmol) dans de la NMP anhydre (3 ml) de la triphényl arsine (1,9 mg ; 0,006 mmol) et du complexe Pd₂(dba)₃ (5,5 mg ; 0,006 mmol). Après 10 minutes d'agitation, une solution du dérivé stannique obtenu à l'exemple 3 (3c) (90 mg ; 0,224 mmol) dans de la NMP (1,5 ml) est ajoutée et le mélange résultant est agité à température ambiante durant 5 heures. Après addition de 4 ml d'une solution aqueuse saturée de KF, on extrait le milieu à l'éther (3 x 5 ml) et les phases organiques rassemblées sont lavées à l'eau (3 x 5 ml), séchées sur MgSO₄ et évaporées. Le résidu est purifié par chromatographie flash sur silice à l'aide de l'éluant hexane/acétate d'éthyle (95/5) pour conduire à 65 mg (90 %) du composé attendu sous forme d'un solide jaune de point de fusion : 121°C.
^{**1**}**H RMN** (250,13 MHz, CDCl₃) δ 1,33 (s, 6H, 2 x CH₃), 1,38 (s, 9H, *t*Bu), 1,94 (t, *J =* 7,1 Hz, 2H, 2H₂,), 2,26 (s, 3H, CH₃), 2,91 (t, *J* = 7,1 Hz, 2H, 2H₃,), 3,79 (s, 3H, CO₂CH₃), 5,93 (d, *J* = 15,3 Hz, 1H, H₂), 6,29 (d, *J* = 11,4 Hz, 1H, H₆), 6,40 (dd, *J* = 14,7, 11,4 Hz, 1H, H₄), 7,01 (dd, *J* = 14,7, 11,4 Hz, 1H, H₅), 7,15 (br s, 2H, 2 x Ar-H), 7,48 (dd, *J* = 15,3, 11,4 Hz, H₃) ppm ;
^{**13**}**C RMN** (62,89 MHz, CDCl₃) δ 17,9 (q), 28,5 (q, 2x), 29,6 (t), 31,5 (q, *t*Bu), 34,6 (s), 41,6 (t), 43,8 (s), 51,3 (q), 118,2 (d), 119,6 (d), 122,2 (d), 128,3 (d), 129,6 (d), 136,9 (s), 137,3 (d), 139,9 (s), 143,6 (s), 145,2 (d), 149,9 (s), 153,1 (s), 167,6 (s) ppm ;
**FTIR** (CHCl₃) **ν** 3026 (w, C-H), 2956 (s, C-H), 2865 (m, C-H), 1715 (s, C=O), 1606 (s, C=C), 1440 (m), 1359 (w), 1308 (m), 1252 (m), 1139 (m), 1001 (m), 881 (w) cm⁻¹.

### EXEMPLE 8 : Acide (2E,4E,6E)-7-[4-(6-tert-butyl-1,1-diméthylindanyl)]-7-méthylhepta-2,4,6-triènoïque

Une solution de l'ester obtenu ci-dessus à l'exemple **7** (57 mg ; 0,162 mmol) dans 0,5 ml d'éther est ajoutée, goutte à goutte, à une solution sous agitation de KOSiMe₃ (90 %,23 mg ; 0,162 mmol) dans de l'éther (1 ml) et le milieu réactionnel est agité pendant 12h à 40°C. Après addition de 2 ml d'eau et agitation pendant 1 heure à température ambiante, le pH de la solution est ajusté à 4-5 par addition d'HCl 0,1N. La phase aqueuse est alors extraite à l'éther (5 x 5 ml) et les phases organiques rassemblées sont séchées sur MgSO₄, filtrées et concentrées sous vide. Le résidu est purifié par chromatographie flash sur silice, à l'aide de l'éluant hexane/acétate d'éthyle (80/20) pour conduire à 40 mg (73 %) de l'acide sous forme d'un solide jaune, de point de fusion : 196°C.
^{**1**}**H RMN** (250,13 MHz, CDCl₃) δ 1,28 (s, 6H, 2 x CH₃), 1,34 (s, 9H, tBu), 1,91 (t, *J* = 7,0 Hz, 2H, 2H₂,), 2,24 (s, 3H, CH₃), 2,88 (t, *J* = 7,0 Hz, 2H, 2H₃), 5,90 (d, *J* = 15,1 Hz, 1H, H₂), 6,28 (d, *J* = 11,5 Hz, 1H, H₆), 6,40 (dd, *J* = 14,2, 11,5 Hz, 1H, H₄), 7,03 (dd, *J* = 14,7, 11,2 Hz, 1H, H₅), 7,12 (m, 2H, 2 x Ar-H), 7,53 (dd, *J* = 15,1, 11,5 Hz, H₃) ppm ;
^{**13**}**C RMN** (62,89 MHz, CDCl₃) δ 18,0 (q), 28,5 (q, 2x), 29,7 (t), 31,6 (q, *t*Bu), 34,7 (s), 41,7 (t), 43,8 (s), 118,3 (d), 119,2 (d), 122,2 (d), 128,3 (d), 129,4 (d), 136,3 (d), 137,0 (s), 138,3 (d), 139,9 (s), 147,3 (s), 149,9 (s), 153,2 (s), 172,4 (s) ppm ;
**FTIR** (CHCl₃) ν 3051 (m, C-H), 2956 (s, C-H), 2857 (m, C-H), 1679 (s, C=O), 1592 (s, C=C), 1447 (m), 1259 (m), 1188 (m), 960 (w), 757 (w) cm⁻¹.

### EXEMPLE 9 : Dans cet exemple on a illustré diverses formulations pharmaceutiques et cosmétiques à base des composés selon l'invention

### A) VOIE ORALE

### (a) On prépare la composition suivante sous la forme d'un comprimé à 0,8 g

- Composé de l'exemple 1 0,005 g
- Amidon prégélatinisé 0,265 g
- Cellulose microcristalline 0,300 g
- Lactose 0,200 g
- Stéarate de magnésium 0,030 g

Pour le traitement de l'acné, on administre à un individu adulte 1 à 3 comprimés par jour pendant 3 à 6 mois selon la gravité du cas traité.

### (b) On prépare une suspension buvable, destinée à être conditionnée en ampoules de 5 ml :

- Compose de l'exemple 2 0,050 g
- Glycérine 0,500 g
- Sorbitol 70 % 0,500 g
- Saccharinate de sodium 0,010 g
- Parahydroxybenzoate de méthyle 0, 040 g
- Arôme q.s.
- Eau purifiée q.s.p. 5 ml

Pour le traitement de l'acné, on administre à un individu adulte 1 ampoule par jour pendant 3 mois selon la gravité du cas traité.

### (c) On prépare la formulation suivante destinée à être conditionnée en gélules :

- Compose de l'exemple 4 0,025 g
- Amidon de maïs 0,060 g
- Lactose q.s.p. 0,300 g

Les gélules utilisées sont constituées de gélatine, d'oxyde de titane et d'un conservateur.

Dans le traitement du psoriasis, on administre à un individu adulte, 1 gélule par jour pendant 30 jours.

### B) VOIE TOPIQUE

### (a) On prépare une crème eau-dans-l'huile non ionique en procédant au mélange des ingrédients suivants :

- Composé de l'exemple 4 0,100 g
- Mélange d'alcools de lanoline émulsifs, de cires et d'huiles raffinés, vendu sous la dénomination "Eucérine anhydre" par la Société BDF 39,900 g
- Parahydroxybenzoate de méthyle 0,075 g
- Parahydroxybenzoate de propyle 0, 075 g
- Eau déminéralisée stérile q.s.p 100, 000 g

Cette crème est appliquée sur une peau psoriatique 1 à 2 fois par jour pendant 30 jours.

### (b) On prépare un gel en procédant au mélange des ingrédients suivants :

- Composé de l'exemple 5 0,050 g
- Erythromycine base 4,000 g
- Butylhydroxytoluène 0,050 g
- Hydroxypropylcellulose vendue sous la dénomination de "KLUCEL HF" par la société Hercules 2,000 g
- Ethanol (à 95°) q.s.p. 100,000 g

Ce gel est appliqué sur une peau atteinte de dermatose ou une peau acnéique 1 à 3 fois par jour pendant 6 à 12 semaines selon la gravité du cas traité.

### (c) On prépare une lotion antiséborrhéique en procédant au mélange des ingrédients suivants :

- Composé de l'exemple 6 0,030 g
- Propylène glycol 5,000 g
- Butylhydroxytoluène 0,100 g
- Ethanol (à 95°) q.s.p. 100,000 g

Cette lotion est appliquée deux fois par jour sur un cuir chevelu séborrhéique et on constate une amélioration significative dans un délai compris entre 2 et 6 semaines.

### (d) On prépare une composition cosmétique contre les effets néfastes du soleil en procédant au mélange des ingrédients suivants :

- Composé de l'exemple 7 1,000 g
- Benzylidène camphre 4,000 g
- Triglycérides d'acides gras 31, 000 g
- Monostéarate de glycérol 6,000 g
- Acide stéarique 2,000 g
- Alcool cétylique 1,200 g
- Lanoline 4,000 g
- Conservateurs 0,300 g
- Propylène glycol 2,000 g
- Triéthanolamine 0,500 g
- Parfum 0,400 g
- Eau déminéralisée q.s.p. 100,000 g

Cette composition est appliquée quotidiennement, elle permet de lutter contre le vieillissement photoinduit.

### (e) On prépare une crème huile-dans-l'eau non ionique en procédant au mélange des ingrédients suivants :

- Compose de l'exemple 8 0,500 g
- Vitamine D₃ 0,020 g
- Alcool cétylique 4,000 g
- Monostéarate de glycérol 2,500 g
- Stéarate de polyéthylèneglycol 50 2,500 g
- Beurre de Karité 9,200 g
- Propylène glycol 2,000 g
- Parahydroxybenzoate de méthyle 0,075 g
- Parahydroxybenzoate de propyle 0,075 g
- Eau déminéralisée stérile q.s.p. 100,000 g

Cette crème est appliquée sur une peau psoriatique 1 à 2 fois par jour pendant 30 Jours.

### (f) On prépare un gel topique anti-acnéique en procédant au mélange des ingrédients suivants :

- Composé de l'exemple 4 0,100 g
- Poloxamer 182 (Pluronic L 62) 0,200 g
- Propylène glycol 4,000 g
- Polymère carboxyvinylique vendu sous la dénomination de "Carbopol 940" par la Société "Goodrich" 0,800 g
- Acide éthylène diamine tétracétique (EDTA) disodique 0,100 g
- Parahydroxybenzoate de méthyle 0,100 g
- NaOH 10 % dans eau 1,250 g
- Eau déminéralisée stérile q.s.p. 100,000 g

Ce gel est appliqué dans le traitement de l'acné 1 à 3 fois par jour pendant 6 à 12 semaines selon la gravité du cas traité.

### (g) On prépare une lotion capillaire anti-chute et pour la repousse des cheveux en procédant au mélange des ingrédients suivants :

- Composé de l'exemple 3 0,05 g
- Composé vendu sous la dénomination de "Minoxidil" 1,00 g
- Propylène glycol 20,00 g
- Ethanol 34,92 g
- Polyéthylèneglycol (masse moléculaire = 400) 40,00 g
- Butylhydroxyanisole 0,01 g
- Butylhydroxytoluène 0,02 g
- Eau q.s.p. 100,00 g

On applique cette lotion 2 fois par jour pendant 3 mois sur un cuir chevelu ayant subi une chute de cheveu importante.

### (h) On prépare une crème anti-acnéique en procédant au mélange des ingrédients suivants :

- Composé de l'exemple 4 0,050 g
- Acide rétinoïque 0,010 g
- Mélange de stéarates de glycérol et de polyéthylène glycol (75 moles) vendu sous la dénomination de "Gelot 64" par la Société "GATTEFOSSE" 15,000 g
- Huile de noyau polyoxyéthylénée à 6 moles d'oxyde d'éthylène vendue sous la dénomination de "Labrafil M2130 CS" par la Société "GATTEFOSSE" 8,000 g
- Perhydrosqualène 10,000 g
- Conservateurs q.s.
- Polyéthylèneglycol (masse moléculaire = 400). 8,000 g
- Sel disodique de l'acide éthylène-diamine tétracétique 0,050 g
- Eau purifiée q.s.p. 100,000 g

Cette crème est appliquée sur une peau atteinte de dermatose ou une peau acnéique 1 à 3 fois par jour pendant 6 à 12 semaines.

### (i) On prépare une crème huile-dans-l'eau en procédant au mélange des ingrédients suivants :

- Composé de l'exemple 5 0,020 g
- 17-Valérate de bétaméthasone 0,050 g
- S-carboxyméthyl cystéine 3,000 g
- Stéarate de polyoxyéthylène (40 moles d'oxyde d'éthylène) vendu sous la dénomination de "Myrj 52" par la Société "ATLAS" 4,000 g
- Monolaurate de sorbitan polyoxyéthylèné à 20 moles d'oxyde d'éthylène vendu sous la dénomination de "Tween 20" par la société "ATLAS" 1,800 g
- Mélange de mono et de distéarate de glycérol vendu sous la dénomination de "Géléol" par la société "GATTEFOSSE" 4,200 g
- Propylène glycol 10,000 g
- Butylhydroxyanisole 0,010 g
- Butylhydroxytoluène 0,020 g
- Alcool cétylstéarylique 6,200 g
- Conservateurs q.s.
- Perhydrosqualène 18,000 g
- Mélange de triglycérides caprylique-caprique vendu sous la dénomination de "Miglyol 812" par la Société "DYNAMIT NOBEL" 4,000 g
- Triéthanolamine (99 % en poids) 2,500 g
- Eau q.s.p. 100,000 g

Cette crème est appliquée 2 fois par jour sur une peau atteinte de dermatose pendant 30 jours.

### (j) On prépare une crème de type huile-dans-l'eau en procédant au mélange des ingrédients suivants :

- Acide lactique 5,000 g
- Composé de l'exemple 2 0,020 g
- Stéarate de polyoxyéthylène (40 moles d'oxyde d'éthylène) vendu sous la dénomination de "Myrj 52" par la société "ATLAS" 4,000 g
- Monolaurate de sorbitan polyoxyéthylèné à 20 moles d'oxyde d'éthylène vendu sous la dénomination de "Tween 20" par la société "ATLAS" 1,800 g
- Mélange de mono et de distéarate de glycérol vendu sous la dénomination de "Géléol" par la société "GATTEFOSSE" 4,200 g
- Propylène glycol 10,000 g
- Butylhydroxyanisole 0,010 g
- Butylhydroxytoluène 0,020 g
- Alcool cétostéarylique 6,200 g
- Conservateurs q.s.
- Perhydrosqualène 18,000 g
- Mélange de triglycérides caprylique-caprique vendu sous la dénomination de "Miglyol 812" par la société "DYNAMIT NOBEL". 4,000 g
- Eau q.s.p. 100,000 g

Cette crème est appliquée 1 fois par jour. Elle aide à lutter contre le vieillissement qu'il soit photoinduit ou chronologique.

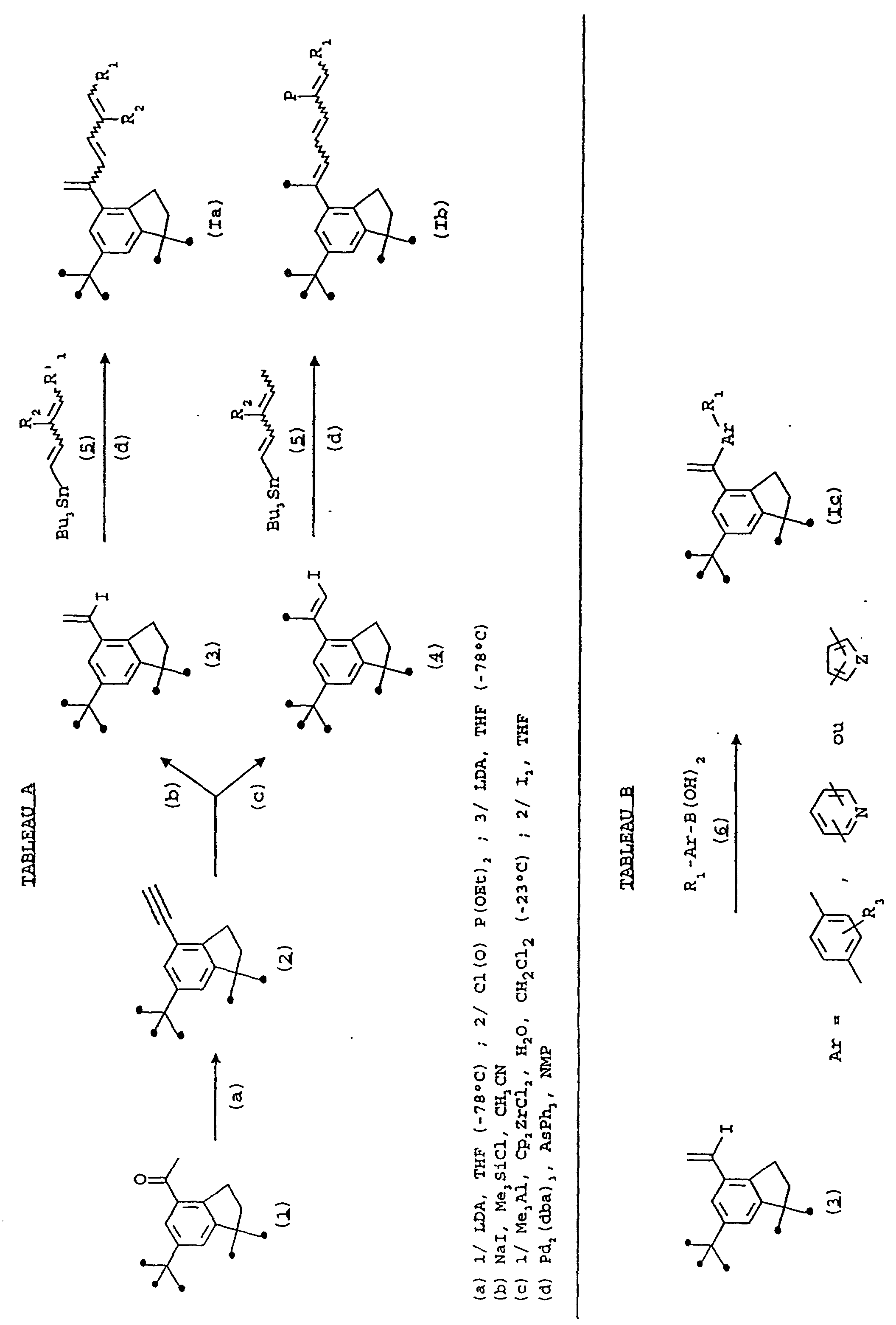

## Revendications

1. Dérivés insaturés en position -4 du 6-*tert*-butyl-1,1-diméthylindane, caractérisés par le fait qu'ils répondent à la formule générale suivante : 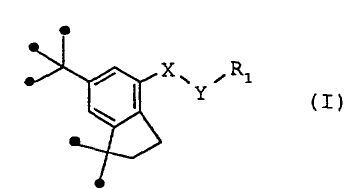 dans laquelle :
X représente :
(i) soit un radical divalent de formule suivante : 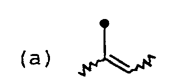 et Y représente alors un radical divalent de formule suivante : 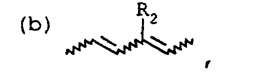
(ii)soit un radical divalent de formule : 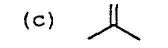 et Y représente alors soit un radical divalent correspondant au radical divalent de formule (b) ci-dessus soit l'un des radicaux divalents de formule suivante : 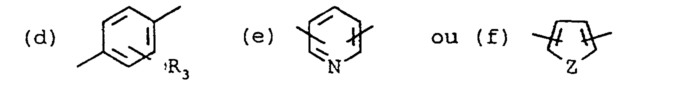
Z étant -O-, -S- ou 〉N-R₃
R₁ représente ―CH₃, - (CH₂)ₚ-OR₄, -(CH₂)ₚ-COR₅, ou -S(O)ₜ-R₆,
p étant 0, 1, 2 ou 3,
t étant 0, 1 ou 2,
R₂ représente H ou alkyle, linéaire ou ramifié, en C₁-C₆,
R₃ représente H, alkoxy en C₁-C₆ ou -OCOR₇,
R₄ représente H, alkyle linéaire ou ramifié en C₁-C₆, ―COR₇, aryle, aralkyle, mono ou polyhydroxyalkyle, ou un radical polyéther,
R₅ représente H, alkyle linéaire ou ramifié en C₁-C₆, ―OR₈ ou 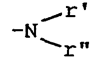
R₆ représente H ou alkyle linéaire ou ramifié en C₁-C₆,
R₇ représente alkyle linéaire ou ramifié en C₁-C₆,
R₈ représente H, alkyle, alkényle, alkynyle, aryle, aralkyle, mono- ou polyhydroxyalkyle, un reste de sucre ou d'acide aminé.
r' et r", identiques ou différents, représentent H, alkyle linéaire ou ramifié en C₁-C₆, ―COR₇, aryle, un reste de sucre, un reste d'acide aminé ou r' et r" pris ensemble, forment un hétérocycle et les sels des composés de formule (I) lorsque R₁ représente une fonction acide carboxylique et les isomères géométriques et optiques des composés de formule (I).

2. Composés selon la revendication 1, caractérisés par le fait qu'ils se présentent sous forme d'un sel d'un métal alcalin ou alcalino-terreux ou encore de zinc ou d'une amine organique.

3. Composés selon l'une des revendications 1 ou 2, caractérisés par le fait que le radical alkyle en C₁-C₆, linéaire ou ramifié, est choisi dans le groupe constitué par les radicaux méthyle, éthyle, propyle, isopropyle, tert-butyle et n-hexyle.

4. Composés selon l'une quelconque des revendications précédentes, caractérisés par le fait que le radical alkyle est un radical en C₁-C₂₀, linéaire ou ramifié, choisis parmi les radicaux méthyle, éthyle, isopropyle, butyle, tert-butyle, 2-éthyl-hexyle, octyle, dodécyle, hexadécyle et octadécyle.

5. Composés selon l'une quelconque des revendications précédentes, caractérisés par le fait que le radical alkényle est un radical en C₂-C₅ présentant une ou deux insaturation(s) éthylénique(s) tel que le radical allyle.

6. Composés selon l'une quelconque des revendications précédentes, caractérisés par le fait que le radical alkynyle est un radical en C₃-C₆ choisi parmi les radicaux 2-propynyle, 2-butynyle ou 2,4 hexadiynyle.

7. Composés selon l'une quelconque des revendications précédentes, caractérisés par le fait que le radical acyle de formule ―COR₇ (R₇ étant alkyle linéaire ou ramifié, en C₁-C₆,) est de préférence choisi parmi les radicaux acétyle, propionyle ou pivaloyle.

8. Composés selon l'une quelconque des revendications précédentes, caractérisés par le fait que le radical monohydroxyalkyle est choisi parmi les radicaux 1-hydroxyéthyle, 2-hydroxypropyle, 3-hydroxypropyle ou 4-hydroxybutyle.

9. Composés selon l'une quelconque des revendications précédentes, caractérisés par le fait que le radical polyhydroxyalkyle est choisi parmi les radicaux 2,3-dihydroxypropyle, 2,3,4-trihydroxybutyle, 2,3,4,5-tétrahydroxypentyle ou le reste du pentaérythritol.

10. Composés selon l'une quelconque des revendications précédentes, caractérisés par le fait que le radical polyéther est choisi parmi les radicaux méthoxyméthyléther, méthoxyéthoxyméthyléther ou méthylthiométhyléther.

11. Composés selon l'une quelconque des revendications précédentes, caractérisés par le fait que le radical aryle est un radical phényle éventuellement substitué par au moins un atome d'halogène, une fonction hydroxyle ou fonction nitro, un groupe méthoxy ou une fonction amine éventuellement substitué.

12. Composés selon l'une quelconque des revendications précédentes, caractérisés par le fait que le radical aralkyle est le radical benzyle ou phénéthyle éventuellement substitué par au moins un atome d'halogène, un hydroxyle ou une fonction nitro ou un groupe méthoxy.

13. Composés selon l'une quelconque des revendications précédentes, caractérisés par le fait que le reste d'acide aminé est choisi parmi les restes dérivant de la lysine, de la glycine ou de l'acide aspartique.

14. Composés selon l'une quelconque des revendications précédentes, caractérisés par le fait que le reste de sucre est choisi parmi les restes dérivant du glucose, du galactose, du mannose ou de l'acide glucuronique.

15. Composés selon l'une quelconque des revendications précédentes, caractérisés par le fait que l'hétérocycle est choisi parmi les radicaux pipéridino, morpholino, pyrrolidino ou pipérazino, ce dernier étant éventuellement substitué en position 4 par un radical alkyle en C₁-C₆ ou un mono ou polyhydroxyalkyle.

16. Composés selon l'une quelconque des revendications précédentes, caractérisés par le fait qu'ils répondent à la formule (II) suivante : 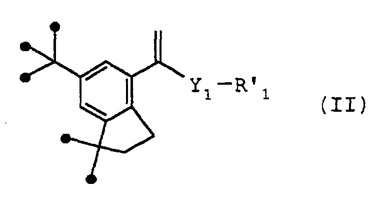 dans laquelle :
Y₁ représente l'un des radicaux divalents de formule : 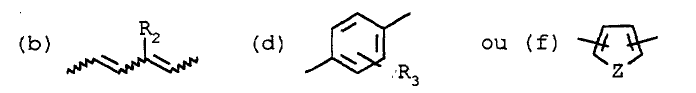
R₂, R₃ et Z ayant les mêmes significations que celles données à la revendication 1,
R'₁ représente - (CH₂)ₚ,―COOR'₈, p' étant 0 et R'₈ représentant H ou alkyle linéaire ou ramifié en C₁-C₆.

17. Composés selon l'une quelconque des revendications 1 à 15, caractérisés par le fait qu'ils répondent à la formule (III) suivante : 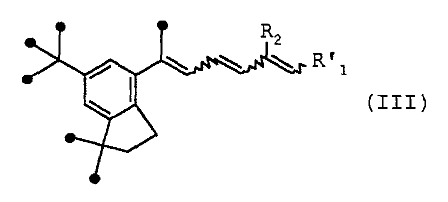 dans laquelle :
R₂ représente H ou alkyle linéaire ou ramifié, en C₁-C₆, et
R'₁ représente -(CH₂ )_{p'}―COOR'₈, p' étant 0 et R'₈ étant H ou alkyle linéaire ou ramifié en C₁-C₆.

18. Composés selon l'une quelconque des revendications précédentes, caractérisés par le fait qu'ils sont choisis dans le groupe constitué par :
- (2E,4E)-6-[4-(6*-tert*-butyl-1,1-diméthylindanyl)]-3-méthylhepta-2,4,6-triènoate de méthyle,
- Acide (2E,4E)-6-[4-(6*-tert*-butyl-1,1-diméthyl-indanyl)]-3-méthylhepta-2,4,6-triènoïque,
- (2E,4E)-6-[4-(6*-tert*-butyl-1,1-diméthylindanyl)]-hepta-2,4,6-triènoate de méthyle,
- Acide (2E,4E)-6-[4-(6*-tert*-butyl-1,1-diméthylindanyl)]-hepta-2,4,6-triènoïque,
- (2E,4E,6E)-7-[4-(6*-tert*-butyl-1,1-diméthylindanyl)]-3,7-diméthylhepta-2,4,6-triènoate de méthyle,
- Acide (2E,4E,6E)-7-[4-(6*-tert*-butyl-1,1-diméthylindanyl)]-3,7-diméthylhepta-2,4,6-triènoïque,
- (2E,4E,6E)-7-[4-(6*-tert*-butyl-1,1-diméthylindanyl)]-7-méthylhepta-2,4,6-triènoate de méthyle,
- Acide (2E,4E,6E)-7-[4-(6*-tert*-butyl-1,1-diméthylindanyl)]-7-méthylhepta-2,4,6-triènoïque,
- 4-[1-[4-(6-*tert*-butyl-1,1-diméthylindanyl)]éthènyl]benzoate d'éthyle,
- Acide 4-[1-[4-(6*-tert*-butyl-1,1-diméthylindanyl)]éthènyl]benzoïque, et
- Acide 5-[1-[4-(6*-tert*-butyl-1,1-diméthylindanyl)]éthènyl]-2-thiophènecarboxylique.

19. Composés selon l'une quelconque des revendications précédentes pour une utilisation comme médicament.

20. Composés selon la revendication 19 pour une utilisation comme médicament destiné au traitement des affections dermatologiques, des affections dermatologiques à composante inflammatoire et/ou immunoallergique du type rhumatismale ou respiratoire, des affections cardio-vasculaires et ophtalmologiques.

21. Utilisation de l'un au moins des composés tels que définis selon l'une quelconque des revendications 1 à 18 pour la préparation d'un médicament destiné au traitement des affections dermatologiques, des affections dermatologiques à composante inflammatoire et/ou immunoallergique du type rhumatismale ou respiratoire, des affections cardio-vasculaires et des troubles ophtalmologiques.

22. Composition pharmaceutique, caractérisée par le fait qu'elle comprend, dans un support pharmaceutiquement acceptable, au moins un composé tel que défini selon l'une quelconque des revendications 1 à 18.

23. Composition selon la revendication 22, caractérisée en ce que la concentration en au moins un composé selon l'une des revendications 1 à 18 est comprise entre 0,001 % et 5 % en poids par rapport au poids total de la composition.

24. Composition cosmétique, caractérisée par le fait qu'elle contient, dans un support cosmétiquement acceptable, au moins un composé tel que défini selon l'une quelconque des revendications 1 à 18.

25. Composition selon la revendication 24, caractérisée en ce que la concentration en au moins un composé selon l'une quelconque des revendications 1 à 18 est comprise entre 0,001 et 3 % en poids par rapport au poids total de la composition.

26. Utilisation d'une composition cosmétique telle que définie selon l'une quelconque des revendications 24 ou 25 pour l'hygiène corporelle ou capillaire.

## Claims

1. 6-tert-Butyl-1,1-dimethylindane derivatives which are unsaturated in the 4-position, characterized in that they correspond to the following general formula: 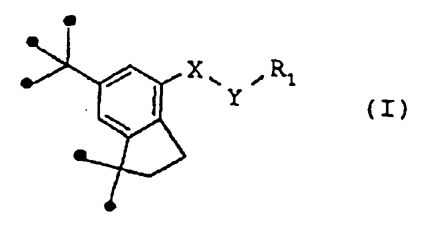 in which:
X represents:
(i) either a divalent radical of following formula: 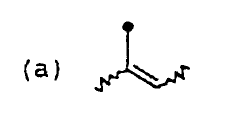 and Y then represents a divalent radical of following formula: 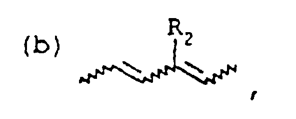
(ii) or a divalent radical of formula: 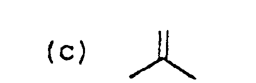 and Y then represents either a divalent radical corresponding to the divalent radical of formula (b) above or one of the divalent radicals of following formula: 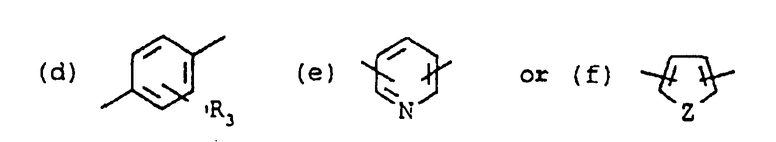
Z being -O-, -S- or 〉N-R₃
R₁ represents -CH₃, -(CH₂)ₚ-OR₄, -(CH₂)ₚ-COR₅ or -S(O)ₜ-R₆,
p being 0, 1, 2 or 3,
t being 0, 1 or 2,
R₂ represents H or linear or branched C₁-C₆ alkyl,
R₃ represents H, C₁-C₆ alkoxy or -OCOR₇,
R₄ represents H, linear or branched C₁-C₆ alkyl, -COR₇, aryl, aralkyl, mono- or polyhydroxyalkyl, or a polyether radical,
R₅ represents H, linear or branched C₁-C₆ alkyl, -OR₈ or 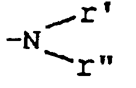
R₆ represents H or linear or branched C₁-C₆ alkyl,
R₇ represents linear or branched C₁-C₆ alkyl,
R₈ represents H, alkyl, alkenyl, alkynyl, aryl, aralkyl, mono- or polyhydroxyalkyl, a sugar residue or an amino acid residue,
r' and r", which are identical or different, represent H, linear or branched C₁-C₆ alkyl, -COR₇, aryl, a sugar residue or an amino acid residue or r' and r", taken together, form a heterocycle, and the salts of the compounds of formula (I), when R₁ represents a carboxylic acid functional group, and the geometrical and optical isomers of the compounds of formula (I).

2. Compounds according to Claim 1, characterized in that they are provided in the form of a salt of an alkali metal or alkaline earth metal or alternatively of zinc or of an organic amine.

3. Compounds according to either of Claims 1 and 2, characterized in that the linear or branched C₁-C₆ alkyl radical is chosen from the group consisting of the methyl, ethyl, propyl, isopropyl, tert-butyl and n-hexyl radicals.

4. Compounds according to any one of the preceding claims, characterized in that the alkyl radical is a linear or branched C₁-C₂₀ radical chosen from the methyl, ethyl, isopropyl, butyl, tert-butyl, 2-ethylhexyl, octyl, dodecyl, hexadecyl and octadecyl radicals.

5. Compounds according to any one of the preceding claims, characterized in that the alkenyl radical is a C₂-C₅ radical exhibiting one or two ethylenic unsaturation(s), such as the allyl radical.

6. Compounds according to any one of the preceding claims, characterized in that the alkynyl radical is a C₃-C₆ radical chosen from the 2-propynyl, 2-butynyl or 2,4-hexadiynyl radicals.

7. Compounds according to any one of the preceding claims, characterized in that the acyl radical of formula -COR₇ (R₇ being linear or branched C₁-C₆ alkyl) is preferably chosen from the acetyl, propionyl or pivaloyl radicals.

8. Compounds according to any one of the preceding claims, characterized in that the monohydroxyalkyl radical is chosen from the 1-hydroxyethyl, 2-hydroxypropyl, 3-hydroxypropyl or 4-hydroxybutyl radicals.

9. Compounds according to any one of the preceding claims, characterized in that the polyhydroxyalkyl radical is chosen from the 2,3-dihydroxypropyl, 2,3,4-trihydroxybutyl or 2,3,4,5-tetrahydroxypentyl radicals or the pentaerythritol residue.

10. Compounds according to any one of the preceding claims, characterized in that the polyether radical is chosen from the methoxymethyl ether, methoxyethoxymethyl ether or methylthiomethyl ether radicals.

11. Compounds according to any one of the preceding claims, characterized in that the aryl radical is a phenyl radical optionally substituted by at least one halogen atom, one hydroxyl functional group or nitro functional group, one methoxy group or one optionally substituted amine functional group.

12. Compounds according to any one of the preceding claims, characterized in that the aralkyl radical is the benzyl or phenethyl radical, optionally substituted by at least one halogen atom, one hydroxyl or one nitro functional group or one methoxy group.

13. Compounds according to any one of the preceding claims, characterized in that the amino acid residue is chosen from the residues deriving from lysine, from glycine or from aspartic acid.

14. Compounds according to any one of the preceding claims, characterized in that the sugar residue is chosen from the residues deriving from glucose, from galactose, from mannose or from glucuronic acid.

15. Compounds according to any one of the preceding claims, characterized in that the heterocycle is chosen from the piperidino, morpholino, pyrrolidino or piperazino radicals, the latter optionally being substituted in the 4-position by a C₁-C₆ alkyl radical or a mono- or polyhydroxyalkyl.

16. Compounds according to any one of the preceding claims, characterized in that they correspond to the following formula (II): 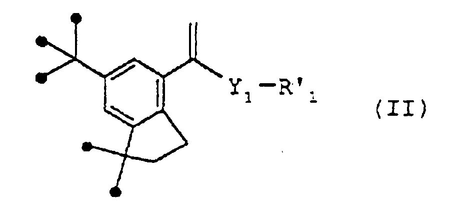 in which:
Y₁ represents one of the divalent radicals of formula: 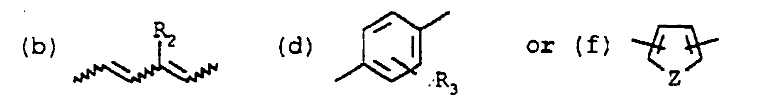
R₂, R₃ and Z having the same meanings as those given in Claim 1,
R'₁ represents - (CH₂)_{p'}-COOR'₈, p' being 0 and R'₈ representing H or linear or branched C₁-C₆ alkyl.

17. Compounds according to any one of Claims 1 to 15, characterized in that they correspond to the following formula (III): 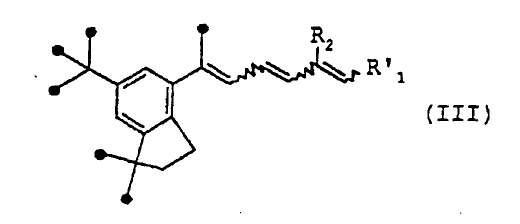 in which:
R₂ represents H or linear or branched C₁-C₆ alkyl, and
R'₁ represents - (CH₂)ₚ,-COOR'₈, p' being 0 and R'₈ being H or linear or branched C₁-C₆ alkyl.

18. Compounds according to any one of the preceding claims, characterized in that they are chosen from the group consisting of:
- methyl (2E,4E)-6-[4-(6-tert-butyl-1,1-dimethylindanyl)]-3-methylhepta-2,4,6-trienoate,
- (2E,4E)-6-[4-(6-tert-butyl-1,1-dimethylindanyl)]-3-methylhepta-2,4,6-trienoic acid,
- methyl (2E,4E)-6-[4-(6*-tert*-butyl-1,1-dimethylindanyl)]hepta-2,4,6-trienoate,
- (2E,4E)-6-[4-(6-*tert*-butyl-1,1-dimethylindanyl)]hepta-2,4,6-trienoic acid,
- methyl (2E,4E,6E)-7-[4-(6-tert-butyl-1,1-dimethylindanyl)]-3,7-dimethylhepta-2,4,6-trienoate,
- (2E,4E,6E)-7-[4-(6-tert-butyl-1,1-dimethylindanyl)]-3,7-dimethylhepta-2,4,6-trienoic acid,
- methyl (2E,4E,6E)-7-[4-(6-*tert*-butyl-1,1-dimethylindanyl)]-7-methylhepta-2,4,6-trienoate,
- (2E,4E,6E)-7-[4-(6-*tert*-butyl-1,1-dimethylindanyl)] -7-methylhepta-2,4,6-trienoic acid,
- ethyl 4-[1-[4-(6-*tert*-butyl-1,1-dimethylindanyl)]ethenyl]benzoate,
- 4-[1-[4-(6*-tert*-butyl-1,1-dimethylindanyl)]-ethenyl]benzoic acid, and
- 5-[1-[4- (6*-tert*-butyl-1,1-dimethylindanyl)]-ethenyl]-2-thiophenecarboxylic acid.

19. Compounds according to any one of the preceding claims for use as medicament.

20. Compounds according to Claim 19 for use as a medicament intended for the treatment of dermatological conditions, dermatological conditions with an inflammatory and/or immunoallergic component of the rheumatic or respiratory type, or cardiovascular and ophthalmological conditions.

21. Use of at least one of the compounds as defined according to any one of Claims 1 to 18 for the preparation of a medicament intended for the treatment of dermatological conditions, dermatological conditions with an inflammatory and/or immunoallergic component of the rheumatic or respiratory type, cardiovascular conditions and ophthalmological disorders.

22. Pharmaceutical composition, characterized in that it comprises, in a pharmaceutically acceptable vehicle, at least one compound as defined according to any one of Claims 1 to 18.

23. Composition according to Claim 22, characterized in that the concentration of at least one compound according to one of Claims 1 to 18 is between 0.001% and 5% by weight with respect to the total weight of the composition.

24. Cosmetic composition, characterized in that it comprises, in a cosmetically acceptable vehicle, at least one compound as defined according to any one of Claims 1 to 18.

25. Composition according to Claim 24, characterized in that the concentration of at least one compound according to any one of Claims 1 to 18 is between 0.001 and 3% by weight with respect to the total weight of the composition.

26. Use of a cosmetic composition as defined according to either one of Claims 24 and 25 for body or hair hygiene.

## Patentansprüche

1. Ungesättigte, in 4-Stellung substituierte 6-tert.-Butyl-1,1-dimethylindan-Derivate, dadurch gekennzeichnet, dass sie der folgenden allgemeinen Formel entsprechen: 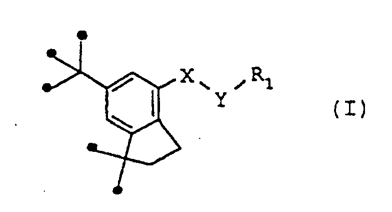 worin:
X darstellt:
(i) entweder einen divalenten Rest der folgenden Formel: 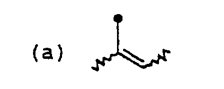 und Y für einen divalenten Rest der folgenden Formel steht: 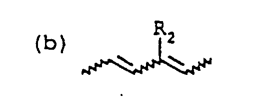
(ii) oder einen divalenten Rest der Formel: 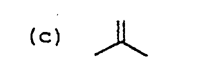 und Y entweder für einen divalenten Rest, entsprechend einem divalenten Rest der obigen Formel (b) oder einen der divalenten Reste der folgenden Formeln steht: 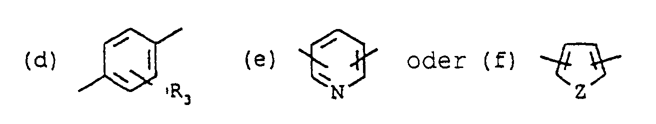
worin
Z -O-, -S- oder 〉N-R₃ ist,
R₁ für -CH₃, - (CH₂)ₚ-OR₄, - (CH₂) ₚ -COR₅ oder -S(O)ₜ-R₆ steht,
p 0, 1, 2 oder 3 ist,
t 0, 1 oder 2 ist,
R₂ für H oder einen linearen oder verzweigten C₁-C₆-Alkylrest steht,
R₃ für H, C₁-C₆-Alkoxy- oder -OCOR₇ steht,
R₄ für H, einen linearen oder verzweigten C₁-C₆-Alkylrest, -COR₇, Aryl, Aralkyl, Mono- oder Polyhydroxyalkyl oder einen Polyetherrest steht,
R₅ für H, einen linearen oder verzweigten C₁-C₆-Alkylrest, -OR₈ oder 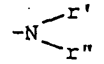 steht,
R₆ für H oder einen linearen oder verzweigten C₁-C₆-Alkylrest steht,
R₇ für einen linearen oder verzweigten C₁-C₆-Alkylrest steht,
R₈ für H, Alkyl, Alkenyl, Alkinyl, Aryl, Aralkyl, Mono-oder Polyhydroxyalkyl, einen Zucker- oder Säureaminrest steht, worin
r' und r", die gleich oder verschieden sind, für H, einen linearen oder verzweigten C₁-C₆-Alkylrest, -COR₇, Aryl, einen Zuckerrest, einen Säureaminrest stehen oder r' und r" zusammengenommen einen Heterozyklus bilden, sowie Salze der Verbindungen der Formel (I), wenn R₁ für eine Carbonsäurefunktion steht, sowie die geometrischen und optischen Isomere der Verbindungen der Formel (I).

2. Verbindungen gemäß Anspruch 1, dadurch gekennzeichnet, dass sie in Form eines Salzes eines Alkalimetalls oder Erdalkalimetalls oder auch von Zink oder einem organischen Amin vorliegen.

3. Verbindungen gemäß den Ansprüchen 1 oder 2, dadurch gekennzeichnet, dass der lineare oder verzweigte C₁-C₆-Alkylrest ausgewählt ist aus der Gruppe, bestehend aus den Methyl-, Ethyl-, Propyl-, Isopropyl-, tert.-Butyl- und n-Hexylresten.

4. Verbindungen gemäß einem der vorstehenden Ansprüche, dadurch gekennzeichnet, dass der Alkylrest ein linearer oder verzweigter C₁-C₂₀-Alkylrest ist, ausgewählt aus Methyl-, Ethyl-, Isopropyl-, Butyl-, tert.-Butyl-, 2-Ethylhexyl-, Octyl, Dodecyl-, Hexadecyl- und Octadecylresten.

5. Verbindungen gemäß einem der vorstehenden Ansprüche, dadurch gekennzeichnet, dass der Alkenylrest ein C₂-C₅-Rest mit einer oder zwei ethylenischen Unsättigungen wie der Allylrest ist.

6. Verbindungen gemäß einem der vorstehenden Ansprüche, dadurch gekennzeichnet, dass der Alkinylrest ein C₃-C₆-Rest ist, ausgewählt unter den Resten 2-Propinyl, 2-Butinyl oder 2,4-Hexadiinyl.

7. Verbindungen gemäß einem der vorstehenden Ansprüche, dadurch gekennzeichnet, dass der Acylrest der Formel -COR₇ (R₇ ist ein linearer oder verzweigter C₁-C₆-Alkylrest) vorzugsweise ausgewählt ist unter den Resten Acetyl, Propionyl oder Pivaloyl.

8. Verbindungen gemäß einem der vorstehenden Ansprüche, dadurch gekennzeichnet, dass der Monohydroxyalkylrest ausgewählt ist unter den Resten 1-Hydroxyethyl, 2-Hydroxypropyl, 3-Hydroxypropyl oder 4-Hydroxybutyl.

9. Verbindungen gemäß einem der vorstehenden Ansprüche, dadurch gekennzeichnet, dass der Polyhydroxyalkylrest ausgewählt ist unter den Resten 2,3-Dihydroxypropyl, 2,3,4-Trihydroxybutyl, 2,3,4,5-Tetrahydroxypentyl oder dem Pentaerythritolrest.

10. Verbindungen gemäß einem der vorstehenden Ansprüche, dadurch gekennzeichnet, dass der Polyetherrest ausgewählt ist unter den Resten Methoxymethylether, Methoxyethoxymethylether oder Methylthiomethylether.

11. Verbindungen gemäß einem der vorstehenden Ansprüche, dadurch gekennzeichnet, dass der Arylrest ein gegebenenfalls mit mindestens einem Halogenatom, einer Hydroxylfunktion oder Nitrofunktion, einer Methoxygruppe oder einer gegebenenfalls substituierten Aminfunktion substituierter Phenylrest ist.

12. Verbindungen gemäß einem der vorstehenden Ansprüche, dadurch gekennzeichnet, dass der Aralkylrest ein gegebenenfalls mit mindestens einem Halogenatom einem Hydroxyl oder einer Nitrofunktion oder einer Methoxygruppe substituierter Benzyl-oder Phenethylrest ist.

13. Verbindungen gemäß einem der vorstehenden Ansprüche, dadurch gekennzeichnet, dass der Säureaminrest ausgewählt ist unter den Resten, die sich von Lysin, dem Glycin oder der Asparaginsäure ableiten.

14. Verbindungen gemäß einem der vorstehenden Ansprüche, dadurch gekennzeichnet, dass der Zuckerrest ausgewählt ist unter den Resten, die sich von Glucose, Galactose, Mannose oder der Glucuronsäure ableiten.

15. Verbindungen gemäß einem der vorstehenden Ansprüche, dadurch gekennzeichnet, dass der Heterozyklus ausgewählt ist unter den Piperidin-, Morpholin-, Pyrrolidin- oder Piperazinresten, wobei letztere gegebenenfalls in Position 4 mit einem C₁-C₆-Alkylrest oder einem Mono- oder Polyhydroxyalkyl substituiert sein können.

16. Verbindungen gemäß einem der vorstehenden Ansprüche, dadurch gekennzeichnet, dass sie der folgenden Formel (II) entsprechen: 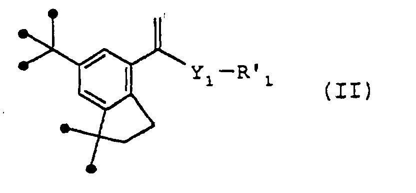 worin:
Y₁ für einen der divalenten Rest der Formel steht: 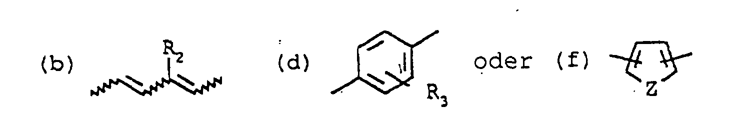
R₂, R₃ und Z dieselbe Bedeutung wie in Anspruch 1 haben,
R'₁ für -(CH₂)_{p'}-COOR'₈ steht, wobei p' 0 ist und R'₈ für H oder ein lineares oder verzweigtes C₁-C₆-Alkyl steht.

17. Verbindungen gemäß einem der Ansprüche 1 bis 15, dadurch gekennzeichnet, dass sie der folgenden Formel (III) entsprechen: 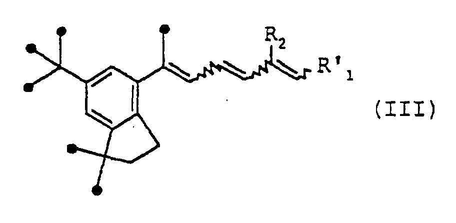 worin:
R₂ für H oder einen linearen oder verzweigten C₁-C₆-Alkylrest steht, und
R'₁ für - (CH₂)ₚ,-COOR'₈ steht, worin p' 0 ist und R'₈ H oder ein linearer oder verzweigter C₁-C₆-Alkylrest ist.

18. Verbindungen gemäß einem der vorstehenden Ansprüche, dadurch gekennzeichnet, dass sie ausgewählt sind aus der Gruppe, bestehend aus:
- (2E,4E)-6-[4-(6-tert.-Butyl-1,1-dimethylindanyl)]-3-methylhepta-2,4,6-trienoatmethylester,
- (2E,4E)-6-[4-(6-tert.-Butyl-1,1-dimethylindanyl)]-3-methylhepta-2,4,6-trienonsäure,
- (2E,4E)-6-[4-(6-tert.-Butyl-1,1-dimethylindanyl)]-hepta-2,4,6-trienoatmethylester,
- (2E,4E)-6-[4-(6-tert.-Butyl-1,1-dimethylindanyl)]-hepta-2,4,6-trienonsäure,
- (2E,4E,6E)-7-[4-(6-tert.-Butyl-1,1-dimethylindanyl)]-3,7-dimethylhepta-2,4,6-trienoatmethylester,
- (2E,4E,6E)-7-[4-(6-tert.-Butyl-1,1-dimethylindanyl)]-3,7-dimethylhepta-2,4,6-trienonsäure,
- (2E,4E,6E)-7-[4-(6-tert.-Butyl-1,1-dimethylindanyl)]-7-methylhepta-2,4,6-trienoatmethylester,
- (2E,4E,6E)-7-[4-(6-tert.-Butyl-1,1-dimethylindanyl)]-7-methylhepta-2,4,6-trienonsäure,
- 4-(1-[4-(6-tert.-Butyl-1,1-dimethylindanyl)]ethenyl)-benzoatethylester,
- 4-(1-[4-(6-tert.-Butyl-1,1-dimethylindanyl)]ethenyl)-benzoesäure, und
- 5-{1-[4-(6-tert.-Butyl-1,1-dimethylindanyl)]ethenyl}-2-thiophencarbonsäure;

19. Verbindungen gemäß einem der vorstehenden Ansprüche zur Verwendung als Medikament.

20. Verbindungen gemäß Anspruch 19 zur Verwendung als Medikament zur Behandlung dermatologischer Leiden, dermatologischer Leiden mit inflammatorischer und/oder immunallergischer Komponente rheumatischer oder respiratorischer Art, von cardiovaskulären und ophtalmologischen Leiden.

21. Verwendung mindestens einer der Verbindungen; wie sie in einem der Ansprüch 1 bis 18 definiert sind, zur Herstellung eines Medikaments zur Behandlung von dermatologischen Leiden, von dermatologischen Leiden mit inflammatorischer und/oder immunallergischer Komponente rheumatischer oder respiratorischer Art, von cardiovaskulären Leiden und ophtalmologischen Beschwerden.

22. Pharmazeutische Zubereitung, dadurch gekennzeichnet, dass sie in einem pharmazeutisch annehmbaren Träger mindestens eine Verbindung enthält, wie sie gemäß einem der Ansprüche 1 bis 18 definiert ist.

23. Zubereitung gemäß Anspruch 22, dadurch gekennzeichnet, dass die Konzentration der mindestens einen Verbindung gemäß einem der Ansprüche 1 bis 18 zwischen 0,001 und 5 Gew.-% liegt, bezogen auf das Gesamtgewicht der Zubereitung.

24. Kosmetische Zubereitung, dadurch gekennzeichnet, dass sie in einem kosmetisch annehmbaren Träger mindestens eine Verbindung enthält, wie sie gemäß einem der Ansprüche 1 bis 18 definiert ist.

25. Zubereitung gemäß Anspruch 24, dadurch gekennzeichnet, dass, dass die Konzentration der mindestens einen Verbindung gemäß einem der Ansprüche 1 bis 18 zwischen 0,001 und 3 Gew.-% liegt, bezogen auf das Gesamtgewicht der Zubereitung.

26. Verwendung einer kosmetischen Zubereitung, wie sie gemäß einem der Ansprüche 24 oder 25 definiert ist, für die Körper- oder Haarhygiene.
